# EUROPEAN PATENT APPLICATION

(11) **EP 2 298 416 A2**
(43) Date of publication of application: **23.03.2011**
(21) Application number: 10185893.4
(22) Date of filing: 11.10.2004
(51) Int. Cl.: A61P 29/00, A61K 9/20, A61K 31/135, A61K 9/16

(54) **Sustained-release tramadol formulations with 24-hour clinical efficacy**

(30) Priority: 10.10.2003 US 510380; 23.04.2004 US 564606
(62) Divisional of application: 04024164.8
(71) Applicant: Labopharm Inc., Laval, Quebec H7V 4B4 (CA)
(72) Inventor: Lenaerts, Vincent, Beaconsfield Québec H9W 5E2 (CA); Ouadji-Njiki, Patricia Laure, Montréal Québec H2C 3A5 (CA); Bacon, Jonathan, 27200, Vernon (FR); Ouzérourou, Rachid, Montréal Québec H1K 1L8 (CA); Gervais, Sonia, Laval Québec H7L 5E8 (CA); Rahmouni, Miloud, Dollars-Des-Ormeaux Québec H9G 2M2 (CA); Smith, Damon, St. Laurent Québec H4R 3A5 (CA); Bouchard, Sylvie, Pierrefonds Québec H8Y 2B8 (CA); Robertson, Sybil, Montréal Québec H3K 2Y4 (CA); Fortier, Louise, Ville Mont-Royal Québec (CA)
(74) Representative: Dossmann, Gérard

(57) **Abstract**

There is disclosed a once daily oral pharmaceutical compositon for controlled release of tramadol or a salt thereof, wherein the composition, when ingested orally, provides a clinical effect over 24 hours which is a least as good as the clinical effect over 24 hours of two doses of a twice daily oral pharmaceutical composition for controlled release of tramadol, taken 12 hours apart.

## Description

### FIELD OF THE INVENTION

This invention relates to a novel once daily oral pharmaceutical composition for controlled release of tramadol or a salt thereof

This invention also relates to a titration kit for rapid titration of a novel once daily oral pharmaceutical composition. This invention further relates to a method of titrating using a novel once daily oral pharmaceutical composition which is capable of rapid titration.

### BACKGROUND OF THE INVENTION

### Tramadol Pharmaceutical Formulations

Tramadol hydrochloride (HCl) was developed by Grünenthal GmbH, Germany It has been marketed in Germany since 1977 (eg. Tramal™), and in the United States as Ultram® since 1995 The efficacy and safety profile of tramadol HCl make it highly suitable as a long-term treatment for chronic pain.

Tramadol HCl is a synthetic, centrally acting analgesic that has been shown to be effective in a variety of acute and chronic pain states. In particular, tramadol HCl, in both immediate and slow-release formulations, in conjunction with non-steroidal anti-infammatory drugs (NSAIDs) (Roth SH. "Efficacy and safety of tramadol HCl In breakthrough musculoskeletal pain attributed to osteoarthritis" J Rheumatol 1998; 25 1358-1363 Wilder-Smith CH et al "Treatment of severe pain from osteoarthritis with slow-release tramadol or dihydrocodeine in combination with NSAID's: a randomized study comparing analgesia, antinociception and gastrointestinal effects" Pain 2001; 91:23-31), has been demonstrated to reduce pain attributed to osteoarthritis (OA) After oral administration, tramedol HCl is rapidly and almost completely absorbed, and it is extensively metabolized. The major metabolic pathways appear to be N- and O-demethylation and glucuronidation or sulfonation in the liver. Only one metabolite, mono-O-desmethyltramadol (M1), is pharmacologically active, which has an approximate 200-fold higher affinity for the µ-opioid receptor than racemic tramadol (DeJong R "Comment on the hypoalgesic effect of tramadol in relation to CYP2D6" (comment) Pain Dig 1997; 7-245; Kogel B et al "Involvement of metabolites in the analgesic action of tramadol" Proc. 9th World Congress on Pain, Vienna, 1999) In healthy humans, tramadol Is demethylated by the polymorphic enzyme cytochrome P450 2D6 (CYP2D6) to the M1 metabolite

The mechanism of action of tramadol HCl is not completely understood Animal models indicate that the drug (and its active M1 metabolite) acts as an opiate agonist, apparently by selective activity at the µ-receptor. In addition to opiate agonist activity, tramadol HCl inhibits re-uptake of certain monoamines (norepinephrine, serotonin) which appears to contribute to the drug's analgesic effect The antinociceptic effect of tramadol HCl is only partially antagonized by naloxone in some tests in animals and humans. In addition, because of the drug's opiate agonist activity, it has been suggested that tramadol HCl may produce dependence; however, its abuse potential appears to be low, and tramadol HCl is not "subject to control" under the United States Federal Controlled Substances Act of 1970 as a scheduled drug.

Immediate release formulations of tramadol HCl are well known in the art. Such formulations, however, require frequent dosing in order to provide effective pain relief Lack of compliance with high frequency dosing regimens can result in inconsistent plasma drug concentrations and accordingly less consistent analgesia. Twice daily formulations are available and are desirable over immediate release formulations as they provide longer periods of analgesia after administration and require less frequent dosing. A once daily formulation is even more desirable for increased effectiveness, safety and convenience.

A critical factor influencing the rate of absorption, and thereby the safety and efficacy, of an active pharmaceutical ingredient by the body following oral administration in a tablet or other solid dosage form is the rate of release of the active pharmaceutical ingredient from that dosage form post ingestion.

it is thus the ability of the dosage form components to control the release rate that constitutes the basis for the so-called controlled-release, extended-release, sustained-release or prolonged-action pharmaceutical preparations that are designed to produce slow, uniform release and absorption of active pharmaceutical ingredients over a period of hours, days, weeks or months. The advantages of such controlled-release formulations include a reduction in the required administration frequency of the drug as compared to conventional immediate release dosage forms, often resulting In improved patient compliance; the maintenance of a stable concentration of the drug in the body and thereby a sustained therapeutic effect over a set period of time; and a decreased incidence and intensity of undesired side effects of the active agent caused by the high plasma concentrations that occur after administration of immediate-release dosage forms

Many materials have been proposed and developed as matrices for the controlled release of active pharmaceutical ingredients. These include, for example, polymeric materials such as polyvinyl chloride, polyethylene amides, ethyl cellulose, silicone and poly (hydroxymethyl methacrylate) See e.g., U S Patent No 3,087,860 to Endicott et al; U S Patent No. 2,987,445 to Levesque et al; Salomon et al Pharm Acta Helv, 55, 174-182 (1980); Korsmeyer, Diffusion Controlled Systems: Hydrogels, Chap 2, pp 15-37 in Polymers for Controlled Drug Delivery, Ed Tarcha, CRC Press, Boca Raton, Fla USA (1991); and Buri et al., Pharm. Acta Helv, 55, 189-197 (1980).

High amylose starch has also been used for controlled-release purposes and, in particular, recent advances have been made using cross-linked high amylose starch For example, United States Patent No. 6,284,273 (Lenaerts et al.), which issued September 4, 2001, and No. 6,419,957 (Lenaerts et al.), which issued July 16, 2002, teach a solid controlled release oral pharmaceutical dosage unit in the form of tablets comprising a dry powder of a pharmaceutical product and a dry powder of cross-iinked high amylose starch, wherein said cross-linked high amylose starch is a matrix comprising a mixture of about 10-60% by weight of amylopectin and about 40-90% amylose. United States Patent No. 6,607,748 (Lenaerts et al) which issued on August 19, 2003 describes a process for making a cross-linked high amylose starch which is known under the name Contramid^{®}.

### Extended Release Formulations Known In the Art

Extended and controlled release formulations relating to tramadol HCl have been suggested, examples being described in: United States Patent Application Publication No 2003/0143270, (Deboeck et al.) published July 31, 2003; United States Patent No. 6,254,887 (Miller et al.) issued July 3, 2001; United States Patent Application Publication No 2001/0036477 (Miller et al) published November 1, 2001; United States Patent No. 6,326,027 (Miller et al) issued December 4, 2001; United States Patent No. 5,591,452 (Miller et al) issued January 7, 1997; European Patent No 1 190 712 (Vanderbist) published March 27, 2002; and WO 03/07205 published September 4, 2003 (Biovail Laboratories Inc.

Although there are some controlled release tramadol HCl formulations on the market which purport to be once-daily formulations, none of these has successfully replaced twice-daily tramadol HCl formulations

Articles have been published in which comparative data between putative "once-daily" tramadol HCl formulations and immediate release tramadol HCl formulations are presented: Adler et al., "A Comparison of Once-Daily Tramadol with Normal Release Tramadol in the Treatment of Pain in Osteoarthritis," The Journal of Rheumatology (2002) 29(10) 2195-2199; and Bodalia et al., "A Comparison of the Pharmacokinetics, Clinical Efficacy, and Tolerability of Once-Daily Tramadol Tablets with Normal Release Tramadol Capsules," Journal of Pain and Symptom Management (2003) 25(2) 142-149.

### Adverse Events from Administration of Tramadol HCl

The most frequently reported side effects of tramadol observed in clinical trials in the United States are constipation, nausea, dizziness/vertigo, headache, somnolence and vomiting. These are typical adverse effects of opiate drugs. Seizures and anaphylactoid reactions have also been reported, though the estimated incidence of seizures in patients receiving tramadol HCl is less than 1% (Kazmierczak, R., and Coley, K. "Doctor letters on prescribing evaluation of the use of tramadol HCl "Formulary 32: 977-978, 1997)

Adler *et al, supra,* reports on the results of a clinical study comparing a once daily tramadol formulation to immediate release tramadol in the treatment of pain in osteoarthritis. The authors report similar adverse event profiles for individuals in both treatment groups Table 2 of Adler *et al*. indicates that a greater percentage of people who were in the once daily treatment group withdrew due to adverse events than did those in the other treatment group

In Bodalia *et al., supra,* the authors report comparable tolerability with a 150 mg once daily dose, a 200 mg once daily dose and three doses of a 50 mg normal release tramadol formulation. This article does not however Include any information on how to make the formulations which are purported to be "once daily" nor does the article disclose any pharmacokinetic data after a single dose.

### Titration

US 6,339,105 (Kamin and Olson) issued January 15, 2002 discloses a regimen for the administration of tramadol for the treatment of analgesia which involves a slow initial titration rate which results in fewer discontinuations of therapy as a result of the incidence and severity of adverse events, compared with more rapid titration rates. The titration regimen provided in US 6,339,105 involves immediate release formulations of tramadol.

The dosage forms for the once daily formulations involved in the clinical study reported in Adler *et al* were 150 mg, 200 mg, 300 mg and 400 mg, and the titration period lasted for 7 to 10 days. Over half of the withdrawals from the study occurred during the titration period as a result of adverse effects

Citation or identification of any reference in this section shall not be construed as an admission that such reference is available as prior art to the present invention.

### SUMMARY OF THE INVENTION

An object of the present invention is to provide an improved sustained-release tramadol formulation with 24-hour effective analgesia

In accordance with one aspect of the present invention, there is provided a once daily oral pharmaceutical composition for controlled release of tramadol or a salt thereof, in which the composition, upon initial administration, provides an onset of analgesic effect within 2 hours, which analgesic effect continues for at least 24 hours after administration.

In accordance with another aspect of the present invention, there is provided a once daily oral pharmaceutical composition for controlled release of tramadol or a salt thereof, wherein the composition, when ingested orally, provides a clinical effect over 24 hours which is at least as good as the clinical effect over 24 hours of two doses of a twice daily oral pharmaceutical composition for controlled release of tramadol, taken 12 hours apart.

In an embodiment of the present invention, there is provided a once daily oral pharmaceutical composition for controlled release of tramadol or a salt thereof, wherein the composition, when ingested orally results in an adverse event profile which is no worse than the adverse event profile of two doses of a twice daily oral pharmaceutical composition for controlled release of tramadol, taken 12 hours apart and provides a clinical effect over 24 hours which is at least as good as the clinical effect over 24 hours of two doses of a twice daily oral pharmaceutical composition for controlled release of tramadol, taken 12 hours apart

In an embodiment of the present invention, there is further provided a method of titrating which comprises administering to one in need thereof about 100 mg of tramadol or a salt thereof, in an oral controlled release pharmaceutical composition, on each of days 1 to 2, about 200 mg of tramadol or a salt thereof, in an oral controlled release pharmaceutical composition on days 3 to 5, about 300 mg of tramadol or a salt thereof, in an oral controlled release pharmaceutical composition on day 6, whereby discontinuations due to adverse events are no greater than those resulting from a less rapid titration

In a further embodiment of the present Invention, there is provided a titration kit comprising a plurality of dosage strengths of a once daily oral pharmaceutical composition comprising tramadol or a salt thereof, wherein there are at least two 100 mg dosage strengths of the composition, at least two 200 mg dosage strength of the composition, and at least one 300 mg dosage strengths of the composition.

The present invention may be understood more fully by reference to the following detailed description and illustrative examples which are intended to exemplify non-limiting embodiments of the invention z

The term "analgesic effect" is defined for the purposes of the present invention as providing a mean blood plasma concentration of at least about 100 ng/mL of tramadol

The term "clinical effect" is defined for purposes of the present Invention as clinical efficacy with respect to pain experienced by study subjects using the WOMAC pain sub-scale score, the WOMAC stiffness and physical function subscale scores and WOMAC global score, a Likert-scale rating of pain at the end of the dosing interval, VAS pain ratings over the 24 hours prior to each visit and patient and investigator global ratings of pain.

The term "controlled release" is defined for purposes of the present invention as a method of oral drug delivery where the rate of release of the active pharmaceutical ingredient from the formulation is not solely dependent on the concentration of active pharmaceutical ingredient remaining in the formulation and/or the solubility of the active pharmaceutical ingredient in the medium surrounding the formulation, and were the time course and/or location of release of an active ingredient from a pharmaceutical formulation are chosen to accomplish therapeutic or convenience objectives not offered by conventional dosage forms.

The term "comparable" is defined for purposes of the present invention as meeting the 80% to 125% limits of acceptability for bioequlvalence.

The term "immediate release" is defined for purposes of the present invention as the release of an active ingredient from a pharmaceutical formulation where the rate of release of the active pharmaceutical ingredient from the pharmaceutical formulation is not retarded by means of a controlled release matrix and where the components of the pharmaceutical formulation are designed such that, upon ingestion, maximum exposure of said active pharmaceutical ingredient to body tissues occurs in the minimum period of time.

The term "initial administration" is defined for purposes of the present invention as the first single dose of a formulation containing an active ingredient administered to a patient or subject or the first dose administered to a patient or subject after a suitable washout period.

The term "mean maximum plasma concentration" (Cₘₐₓ) is defined for the purposes of the present invention as the maximum mean plasma concentration

The term "mean plasma concentration" is defined for purposes of the present invention as the arithmetic mean blood plasma concentration.

The word "tramadol", as used herein shall refer to tramadol, its stereoisomers and its pharmaceutically acceptable salts

The term "steady state" is defined for purposes of the present invention as the state, following multiple dose administration, where the rate of drug elimination matches the rate of input and the plasma drug concentrations at a given time within a dosing interval are approximately the same from one dosing interval to another

The term "twice daily oral pharmaceutical compositon" is defined for purposes of the present invention as a twice-daily formulation supplied by Grünenthal GmbH.

### BRIEF DESCRIPTION OF THE FIGURES

Various features and advantages of the present invention will become clear from the more detailed description given below with reference to the accompanying drawings, in which

**Figure 1****:** Flow diagram showing manufacturing process for tablets.

**Figure 2****:** Dissolution profiles of formulations A, B and C *In vifro* performance of formulations A, B and C under USP Type 1 Conditions; sodium phosphate buffer, 50 mM, pH 6 8, 100 rpm 6 tablets were tested per time point

**Figure 3****:** Mean tramadol plasma concentrations following single-dose administration of (i) a 100 mg dose of the inventive controlled release composition, (ii) a 200 mg dose of the inventive controlled release composition, and (iii) a 300 mg dose of the inventive controlled release composition.

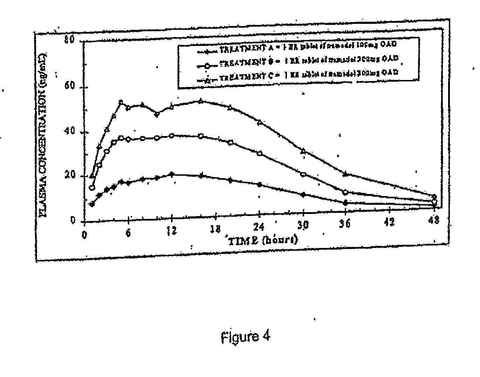

**Figure 4****:** Mean O-desmathyltramadol plasma concentrations of following single dose administration of either 100 mg (◆), 200 mg (○), and 300 mg (Δ) strength tramadol formulations (A, B, and C, respectively).

**Figure 5****:** Mean tramadol plasma concentrations following single-dose administration of (i) 2 x 200 mg doses of the inventive controlled release composition; and (ii) Topalgic® LP 200 mg BID q12h.

**Figure 6****:** Mean O-desmethyltramadol plasma concentrations following single-dose administration of (i) 2 x 200 mg doses of the inventive controlled release composition; and (ii) Topalgic® LP 200 mg BID q12h.

**Figure 7****:** Mean steady-state tramadol and O-desmethyltramadol plasma concentration following administration of (i) a 200 mg dose of the inventive controlled release composition: and (ii) Topalgic® LP 100 mg BID q12h

**Figure 8****:** Phase III Clinical Trial Results WOMAC Pain Sub-scale: improvement (mm) vs. time

**Figure 9****:** Phase III Clinical Trial Results: Daily rating of pain at the end of dosing interval (24 hour efficacy)

**Figure 10****:** Phase III Clinical Trial Results: WOMAC Stiffness Subscale improvement (mm) vs time

**Figure 11****:** Phase III Clinical Trial Results: WOMAC Physical Function Sub-scale improvement (mm) vs time

**Figure 12**: Phase III Clinical Trial Results WOMAC Global Score: improvement (mm) vs time

**Figure 13****:** Phase III Clinical Trial Results Patient Overall Efficacy Rating

**Figure 14****:** Phase III Clinical Trial Results: Physician Overall Efficacy Rating

**Figure 15****:** Phase III Clinical Trial Results Most Common Adverse Events

**Figure 16****:** Steps of titration process.

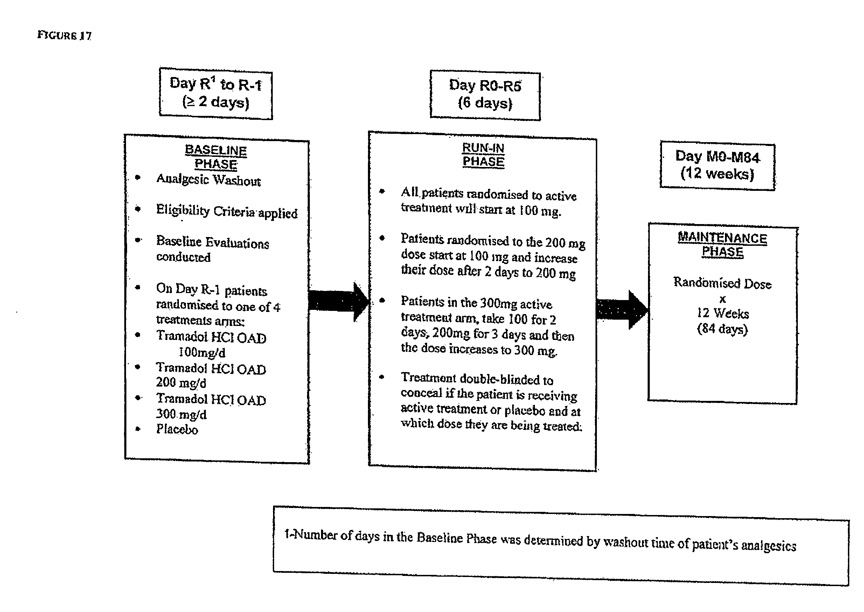

[0054.1] **Figure 17****:** Diagram showing an overview of a first study carried out to compare the analgesic efficacy and safety of tramadol HCl OAD and a placebo for the treatment of pain due to osteoarthritis of the knee.

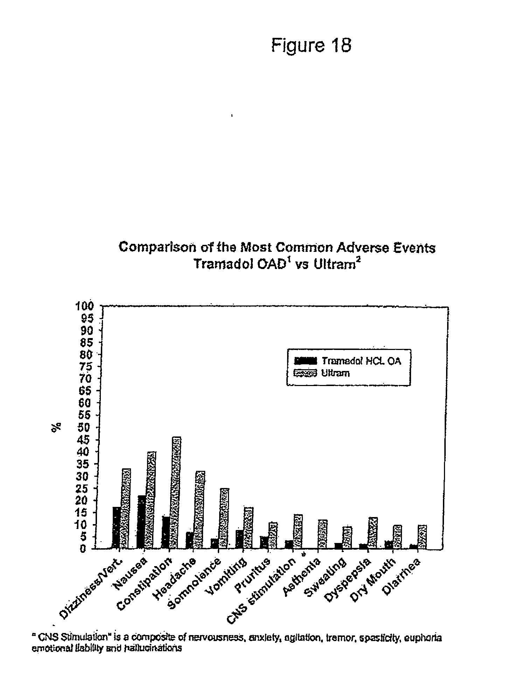

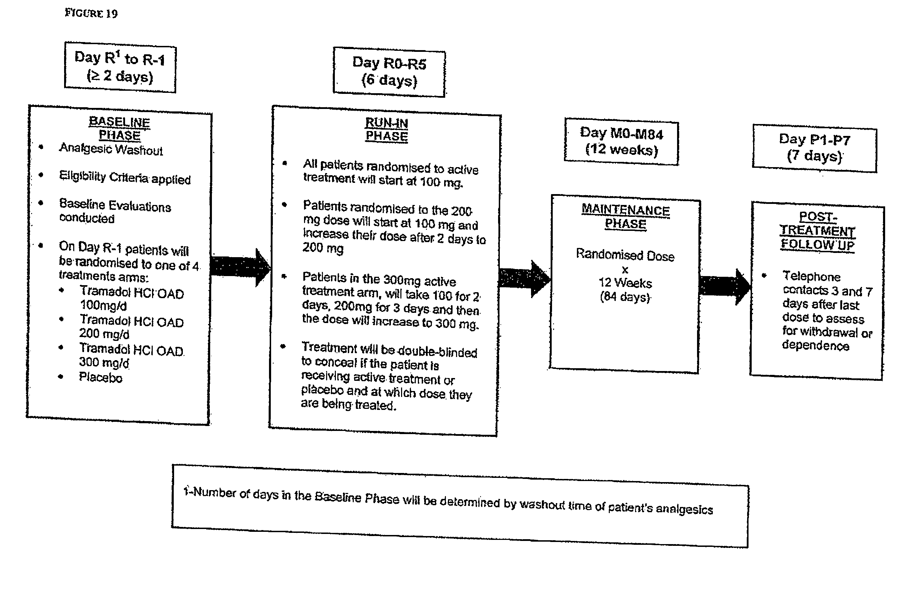

[0054.2] **Figure 18****:** Bar graph showing comparison of adverse events obtained In the first study between a tramadol HCl OAD and Ultram [0054.3] **Figure 19****:** Diagram showing an overview of a second study carried out to compare the analgesic efficacy and safety of tramadol HCl OAD and a placebo for the treatment of pain due to osteoarthritis of the knee.

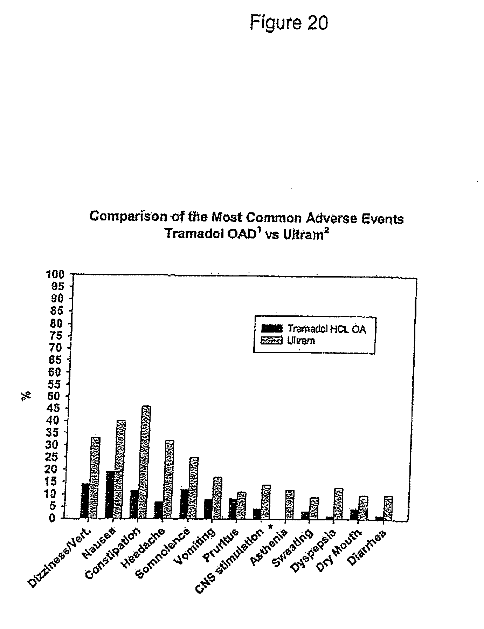

[0054.4] **Figure 20**: Bar graph showing comparison of adverse events obtained in the second study between a tramadol HCl OAD and Ultram

### DETAILED DESCRIPTION OF THE INVENTION

### CORE

The core of a tablet of the invention includes at least one active ingredient and a matrix, these components associated in such a way that release of the pharmaceutical ingredient from the matrix is controlled In a specific embodiment, the matrix of the core is a cross-linked high amylose starch known under the name Contramid^{®}, and described most recently in US Patent No 6,607,748 (Lenaerts et al.), which issued August 19, 2003. A preferred formulation in the context of this invention is provided in the specification of US Patent No 6,607,748

Preferably, the core is formed by admixing the ingredients (in granular or powder form) and then compressing the mixture to form the core over which the coat is subsequently formed The weight of the core can be any percentage of the weight of the total composition between 10% and 80%. The preferred percentage depends, upon other things, the total dosage of the pharmaceutical agent. In a particular embodiment described further below, a tablet contains 100 mg tramadol hydrochloride and the core is about 26% of the total weight of the tablet. In another embodiment a tablet contains 200 mg tramadol hydrochloride and the core makes up about 33% of the total weight of the tablet. In yet another embodiment, a tablet contains 300 mg tramadol hydrochloride, and the core contributes 33% to the total weight of the tablet

### Active Agent in the Core

An active pharmaceutical ingredient is present in the core of the composition of the present invention A suitable pharmaceutical ingredient of the present invention is any such ingredient that is desired to be delivered in a sustained-release dosage form A comprehensive list of suitable pharmaceutical agents can be found in The Merck Index, 12^{th} Ed Preferably, the pharmaceutical ingredient is, but not limited to, isonicotinic acid hydrazide, sodium salicylate, pseudoephedrine hydrochloride, pseudoephedrine sulfate, acetaminophen or diclofenac sodium, verapamil, glipizide, nifedipine, felodipine, betahistine, albuterol, acrivastine, omeprazole, misoprostol, tramadol^{®}, oxybutynin, trimebutine, ciprofloxacin, and salts thereof, In addition, the pharmaceutical agent can be an antifungal agent, such as ketoconazole, or an analgesic agent such as acetylsalicylic acid, acetaminophen, paracetamol, ibuprofen, ketoprofen, indomethacin, diflunisal, naproxen, ketorolac, diclofenac, tolmetin, sulindac, phenacetin, piroxicam, mefamanic acid, dextromethorphan, other non-steroidal anti-inflammatory drugs including salicylates, pharmaceutically acceptable salts thereof or mixtures thereof Pro-drugs are part of the invention

The solubility of the pharmaceutical agent In aqueous solution can be a wide variety of values The aqueous solubility of the pharmaceutical agent can be less than 10⁻³ g/L, more than 10⁻³ g/L, more than 10⁻² g/L, more than 10⁻¹ g/L, more than 1 g/L, more than 10 g/L, more than 100 g/L, more than 500 g/L, more than 1000 g/L, or more than 2000 g/L. Preferably, the solubility is more than 100 g/L, More preferably, the solubility is more than 500 g/L, Most preferably, the solubility is more than 1000 g/L

The pharmaceutical agent can meet a variety of dosage requirement. For example, the dosage requirement of the pharmaceutical agent can be less than 1 mg/dosage unit, more than 1 mg/dosage unit, more than 10 mg/dosage unit, more than 100 mg/dosage unit, more than 200 mg/dosage unit, more than 300 mg/dosage unit, more than 400 mg/dosage unit, more than 500 mg/dosage unit, or more than 1000 mg/dosage unit Preferably, the pharmaceutical agent Is more than 50 mg/dosage unit More preferably, the pharmaceutical agent is 100 mg/dosage unit, or more, e.g 150 mg/dosage unit, or 200 mg/dosage unit, or 250 mg/dosage unit, or 300 mg/dosage unit, or more.

Particular embodiments include a core containing tramadol hydrochloride in which the core contains between about 10% and 90% of the total tramadol present in the tablet, e.g. about 45 mg of a 100 mg strength tablet (45% of the tablet total), or about 90 of a 200 mg strength tablet (45% of the tablet total), or about 151 mg of a 300 mg strength tablet (50% of the tablet total)

### Matrix of the Core

The release from the formulation of an active pharmaceutical ingredient located in the core is slower than the release of an active pharmaceutical ingredient located In the matrix of the coat A preferred matrix of the core is cross-linked high amylose starch, known under the name Contramid^{®} and described in U.S. Patent No. 6,607,748. In particular embodiments, the matrix makes up between about 10% and about 90% by weight of the core i.e., the ratio of the matrix of the core to the active ingredient of the core (w/w) is between about 0.1 and about 10, or between about 0.2 and about 9, or between about 0 2 and about 8, or between about 0 3 and about 7, or between about 0.4 and about 6, or between about 0.5 and about 5, or between about 0.6 and about 4, or between about 0.7 and about 4 or between about 1 and about 4, or between about 1 and about 3 and about 1.5 and about 2.5 In one particular embodiment, the core totals about 90 mg, of which about 44 mg is Contramid^{®}, and 45 mg is tramadol hydrochloride. In this case, Contramid^{®} thus makes up about 49 weight percent of the core.

### Optional Components

The core composition of the present invention may optionally include a pharmaceutically acceptable carrier or vehicle. Such carriers or vehicles are known to those skilled in the art and are found, for example, in Remingtons's Pharmaceutical Science 14th Ed. (1970) Examples of such carriers or vehicles include lactose, starch, dicalcium phosphate, calcium sulfate, kaolin, mannitol and powdered sugar Addifionally, when required, suitable binders, lubricants, and disintegrating agents can be induded. If desired, dyes, as well as sweetening or flavoring agents can be included

The core composition of the present invention may optionally include accessory ingredients including, but not limited to dispersing agents such as microcrystaillne cellulose, starch, cross-linked starch, cross-linked poly(vinyl pyrrolidone), and sodium carboxymethyl cellulose; flavoring agents; coloring agents; binders; preservatives; surfactants and the like

The core can, optionally, also include one or more suitable binders known to one of ordinary skilled in the art.

Suitable forms of microcrystalline cellulose, for example; MCC-PH101, MCG-102, MCC-105, etc.

Suitable lubricants, such as those known to the skilled person, may also be included For example, magnesium stearate, vegetable oil, talc, sodium-stearyl fumarate, calcium stearate, stearic acid, etc.

Suitable glidants, known in the art, may also be included Examples of such glidants include, but are not limited to talc, colloidal silicon dioxide, etc.

### Proportion

The active agent is present at levels ranging from about 1 to about 90 wt % of the total weight of the core, preferably from about 10 to about 70 wt% of the total composition of the core, more preferably from about 20 to about 60 wt% of the total composition of the core, and probably most often between about 30 to about 50 wt.% of the total composition of the core.

Of course, the total amount of all components is 100 wt %, and those of ordinary skill in the art can vary the amounts within the stated ranges to achieve useful compositions.

### COAT

The coat of the dosage form includes a physical mixture of polyvinyl acetate and polyvinylpyrrolidone and the active pharmaceutical ingredient(s) of the coat The coat can also include a cross-linked high amylose starch, e g., Contramid^{®}, and other optional components. In a preferred embodiment, the coat is formed by dry compression The weight of the coat can be any percentage of the weight of the total composition between about 10% and about 90%, but is preferably in the higher part of this range. The coat thus usually makes up between about 20% to about 90%, (w/w) of a tablet of the invention, or about 25% to about 90%, or about 30% to about 85%, or about 35 % to about 85%, or about 40% to about 85%, or about 45% to about 85%, or about 45% to about 90%, or about 50% to about 90% or about 50% to about 85 %, or about 55% to about 90%, or about 55% to about 85%, or about 55% to about 80%, or about 60% to about 90%, or about 60% to about 85%, or about 60% to about 80%, or about 60% to about 75%, or about 65% to about 90%, or about 65% to about 85%, or about 65% to about 80%, or about 65% to about 75%, or about 65% or about 70% or about 75%.
The coat often includes an optional binding agent

### Polyvinyl Acetate and Polyvinylpyrrolidone of the Coat

The weight percentage of the polyvinyl acetat/polyvinylpyrrolidone mixture in the coat can be anywhere within a wide range of values Depending on the solubility in water of the active ingredient in the coat, the amount of the polyvinyl acetate/polyvinylpyrrolidona mixture in the coat can be adjusted. United States Patent Publication No. 2001/0038852 describes ways in which such adjustments can be made. For example, for active ingredients that are soluble to extremely soluble in water, polyvinyl acetate/polyvinylpyrrolidone mixture can be about 20 to about 80 wt.% of the coat, preferably about 30 to about 65 wt.%, or about 40 to about 55 wt.% In a particular embodiment described below, Kollidon^{™} SR makes up about 45% by weight of a coat that is about 31% by weight tramodol hydrochloride and about 23% xanthan gum. For active ingredients that are sparingly soluble to slightly soluble in water, the amount of polyvinyl acetate/polyvinylpyrrolidone mixture is often lower, as described in United States Patent Publication No. 2001/0038852.

The weight ratio of polyvinyl acetate to polyvinylpyrrolidone in the polyvinyl acetate/polyvinylpyrrolidone mixture can be a wide range of values Preferably, such ratio is between about 6:4 and 9.1; more likely between about 73 and 61, even more preferably about 8:2.

The molecular weight of the polyvinyl acetate component in the polyvinyl acetate/polyvinylpyrrolidone mixture can be a wide range of values. Preferably, the average molecular weight of the polyvinyl acetate is about 100 to about 10,000,000; or about 1,000 to about 1,000,000; or about 10,000 to about 1,000,000; or about 100,000 to about 1,000,000; or about 450,000.

The molecular weight of the polyvinylpyrrolidone component in the polyvinyl acetate/polyvinylpyrrolidone mixture can be a wide range of values. The average molecular weight of the polyvinylpyrrolidone can be from about 100 to about 10,000,000; or about 1,000 to about 1,000,000; or about 5,000 to about 500,000; or about 10,000 to about 100,000; or about 50,000

The polyvinyl acetate and polyvinylpyrrolidone mixture can be prepared by a variety of processes including simply mixing powders of polyvinylpyrrolidone and polyvinyl acetate. In a preferred embodiment, such mixture is spray dried powder of a colloidal dispersion of polyvinyl acetate and polyvinylpyrrolidone solution- Optionally, sodium lauryl sulfate is used as a stabilizer in order to prevent agglomeration during spray drying process and/or colloidal silica is used to improve the flow properties of the polyvinyl acetate/polyvinylpyrrolidone mixture. Optionally, polyvinyl acetate and polyvinylpyrrolidone can be formed in a random or a block copolymer

### Optional Components

Suitable binding agents for the present invention include, but are not limited to, plant extracts, gums, synthetic or natural polysaccharides, polypeptides, alginates, synthetic polymers, or a mixture thereof

Sultable plant extracts to be used as gelling agents include, but are not limited to, agar, ispaghula, psyllium, cydonia, ceratonia or a mixture thereof.

Suitable gums to be used as gelling agents include, but are not limited to, xanthan gum, guar gum, acacia gum, ghatti gum, karaya gum, tragacanth gum or a mixture thereof.

Suitable synthetics or natural hydrophilic polysaccharides to be used as gelling agents include, but are not limited to, hydroxyalkylcelluloses, cellulose ethers, cellulose esters, nitrocelluloses, dextrin, agar, carrageenan, pectin, furcellaran, starch or starch derivatives, cross-linked high amylose starch, or a mixture thereof.

Suitable polypeptides to be used as gelling agents include, but are not limited to, gelatin, collegen, polygeline or a mixture thereof

Suitable alginates to be used as gelling agents include, but are not limited to, alginic acid, propylene glycol alginate, sodium alginate or a mixture thereof

Suitable synthetic polymers to be used as gelling agents include, but are not limited to, carboxyvinyl polymer, polyvinyl alcohol, polyvinyl pyrrolidone, polyethelene oxide, polyethylene glycols, copolymers of ethylene oxide and propylene oxide and their copolymers or a mixture thereof.

In a preferred embodiment, the gelling agent is a gum such as xanthan gum, guar gum, acacia gum, ghatti gum, karaya gum, tragacanth gum or a mixture thereof, PEO 7,000,000 and HPMC K100 M

In a most preferred embodiment, the gelling agent is xanthan gum.

### Active agent of the Coat

A suitable active pharmaceutical ingredient of the present invention is any active agent that it is desired to be delivered in a sustained-release dosage form A comprehensive list of suitable pharmaceutical agents can be found in The Merck index, 12^{th} Ed Preferably, the pharmaceutical agent is, but not limited to, isonicotinic acid hydrazide, sodium salicylate, pseudoephedrine hydrochloride, pseudoephedrine sulfate, acetaminophen or diclofenac sodium, verapamil, glipizide, nifedipine, felodipine, betahistine, albuterol, acrivastine, omeprazole, misoprostol, tramadol^{®}, oxybutynin, trimebutine, ciprofloxacin, and salts thereof. In addition, the pharmaceutical agent can be an antifungal agent, such as ketoconazole, or an analgesic agent such as acetylsalicylic acid, acetaminophen, paracetamol, ibuprofen, ketoprofen, indomethacin, diflunisal, naproxen, ketorolac, diclofenac, tolmetin, sulindac, phenacetin, piroxicam, mefamanic acid, dextromethorphan, other non-steroidal anti-inflammatory drugs including salicylates, pharmaceutically acceptable salts thereof or mixtures thereof.

The solubility of the pharmaceutical agent in aqueous solution can be a wide variety of values. The aqueous solubility of the pharmaceutical agent can be less than 10⁻³ g/L, more than 10⁻³ g/L, more than 10⁻² g/L, more than 10⁻¹ g/L, more than 1 g/L, more than 10 g/L, more than 100 g/L, more than 500 g/L, more than 1000 g/L, or more than 2000 g/L Preferably, the solubility is more than 100 g/L. More preferably, the solubility is more than 500 g/L or even 1000 g/L.

The pharmaceutical agent can meet a variety of dosage requirements For example, the dosage requirement of the pharmaceutical agent can be less than 1 mg/dosage unit, more than 1 mg/dosage unit, more than 10 mg/dosage unit, more than 100 mg/dosage unit, more than 200 mg/dosage unit, more than 300 mg/dosage unit, more than 400 mg/dosage unit, more than 500 mg/dosage unit, or more than 1000 mg/dosage unit Preferably, the pharmaceutical agent is more than 50 mg/dosage unit More preferably, the pharmaceutical agent is more than 100 mg/dosage unit Most preferably, the pharmaceutical agent is more than 200 mg/dosage unit

The coat can be between about 5% and about 90% by weight active pharmaceutical ingredient, or between about 5% and about 80% by weight api, or between about 10% and about 70% by weight api, or between about 10% and about 60% by weight api, or between about 15% and about 50% by weight api, or between about 15% and about 45% by weight api, or between about 15% and about 40% by weight api, or between about 20% and about 35% by weight api, or between about 20% and about 30% by weight api

In particular embodiments, described further below, the weight of tramadol from a 100 mg tramadol tablet is about 21% by weight of the coat The weight of tramadol from a 200 mg tablet is about 31 % by weight of the coat The weight of tramadol from a 300 mg tablet is about 30% by weight of the coat.

### ROUTES OF ADMINISTRATION

The tablet composition of the present invention can be administered through, but not limited to, a number of routes such as oral, sublingual, and rectal. The preferred route of administration of the compositions of the present invention is oral.

Compositions of the present invention that are suitable for oral administration may be presented as discrete units such as tablets or granules Preferably, the compositions of the present invention are presented in a tablet form. Such tablets may be conventionally formed by compression or molding. Compressed tablets may be prepared by compressing in a suitable machine the mixture of one or more components described above Molded tablets may be made by molding in a suitable machine the above components, which can be optionally moistened with an Inert liquid diluent. The tablets may optionally be coated and/or have other identifying indicia visible to the consumer. A tablet can also be in a variety of forms, *e.g*, uncoated , dry coated, or film coated, etc. A tablet can also be in a variety of shapes (e.g , oval, sphere, etc.) and sizes. A comprehensive discussion of tablets can be found in references such as The Theory and Practice of Industrial Pharmacy by Lachman et al., 3rd Ed. (Lea & Febiger, 1986)

### Dissolution Profile of Sustained-Release Composition

The active agent of the composition exhibits the following *in vitro* dissolution profile when measured with a USP Type I apparatus in 50 mM phosphate, pH 6 8, and stirring between 50 and 150 rpm an average rate of between 10% and 30% per hour of the agent is released between 0 and 2 hours when tested *in vitro* using a USP Type I apparatus in 50 mM phosphate, pH 6 8, and stirring between 50 and 150 rpm; or
between 10% and 40% of the agent is released from the formulation between 0 and about 2 hours of measurement, between about 30% and 60% of the agent is released from the formulation between 2 and about 7 hours of the measurement, between about 50% and 80% of the agent is released from the formulation between 7 and about 12 hours of measurement, and between about 80% and 100% of the agent is released from the formulation after about 20 hours of measurement; or more preferably
between 15% and 35% of the agent is released from the formulation between at 2 hours of measurement, between about 40% and 60% of the agent is released from the formulation between at 7 hours of the measurement, between about 60% and 80% of the agent is released from the formulation at 12 hours of measurement, and between about 85% and 100% of the agent is released from the formulation after about 20 hours of measurement, or
between 20% and 40% of the agent is released from the formulation between at 2 hours of measurement, between about 40% and 60% of the agent is released from the formulation between at 7 hours of the measurement, between about 60% and 80% of the agent is released from the formulation at 12 hours of measurement, and between about 85% and 100% of the agent is released from the formulation after about 20 hours of measurement.

The present invention will be more readity understood by referring to the following examples which are given to illustrate the invention rather than to limit its scope.

### EXAMPLES

The cross-linked high amylose starch used in the these examples is made by a process comprising the steps of crosslinking and chemically modifying, followed by gelatinization and drying. Such process is described in more detail in U.S Patent No 6,607,748 (Lenaerts et al), which issued August 19, 2003, and known in the marketplace under the name Contramid^{®} and described In Examples 1 and 2

### Example 1

### A. Cross-Linking

High amylose starch (30.0 kg) containing about 70% w/w of amylose (Cl AmyloGel 03003) is placed in a reactor. To this reactor is added water (55,0 1) containing sodium hydroxide (30.0 g) and sodium sulfate (2 40 kg). The resulting slurry is heated to a temperature of 30°C. Phosphorus oxychloride (22.5 g) is added to the reaction mixture which is reacted for one hour.

### B. Chemical Modification, Hydroxyproylation

The crude reaction mixture from Part A is transferred into a hydroxypropylation reactor. The reaction mixture is heated to 40°C- over 30 minutes and the reaction is purged with nitrogen. After a full purge, propylene oxide (1 80 kg) is added The reaction mixture is kept at 40°C, for 20 hours. The reaction mixture is neutralized with 0 1 N H₂SO₄ (1:2 v/v) to a pH of 5 The starch slurry is washed with a basket-centrifuge at a speed of 1200 rpm The obtained starch cake is re-slurrified in 35 I of water and centrifuged a second time. The resulting starch cake is dried in a flash dryer at an inlet temperature of 160°C. and an outlet temperature of 60°C.

### C. Gelatinization

The modified granular starch cake is diluted in demineralized water in order to form a slurry at a concentration of about 8% calculated on dry substance. The resulting slurry has a relative density of 1 032 kg/l compared to water The pH of the modified starch slurry is adjusted to 6 0 The slurry is then heated to 160°C by direct steam injection (Schlick Model 825). The temperature variation is not higher than ±1°C. The slurry is held in a holding column for a period of 4 minutes at a temperature of 160°C. and a pressure of about 5 5 bar. The pressure is then reduced to atmospheric by passing through a flash. The slurry is then contained at 95°C. in a hold tank.

### D. Spray-Drying

The drying of the slurry from Part C is carried out using a Niro FSD 4 spray-drying tower equipped with a 0 8 mm nozzle and fed at 10 l/hour The inlet temperature is fixed at 300°C. and the outlet temperature of 120°C. The obtained powder is a controlled release excipient with the following properties

| Properties | |
|---|---|
| Moisture Content | 4 5% |
| Bulk Density | 150 g/l |
| Packed Density | 210 g/l |
| pH | 54 |
| Particle Size Peak Value | 50 µm |
| (Laser Particle Sizer-Sympatec) | |

### Example 2

### A. Cross-Linking

High amylose starch (30.0 kg) containing about 70% w/w of amylose (Cl AmyloGel 03003) is placed in a reactor. To this reactor is added water (55-01) containing sodium hydroxide (30.0 g) and sodium sulfate (2 40 kg) The resulting slurry is heated to a temperature of 30°C Sodium trimetaphosphate (45 g) is added to the reaction mixture which is reacted for one hour.

### B. Chemical Modification, Hydroxyproylation

The crude reaction mixture from Part A is transferred into a hydroxypropylation reactor The reaction mixture is heated to 40°C over 30 minutes and the reaction is purged with nitrogen. After a full purge, propylene oxide (1 80 kg) is added The reaction mixture is kept at 40°C for 20 hours. The reaction mixture is neutralized with 0.1N H₂SO₄ (1·2 v/v) to a pH of 55 The starch slurry is washed with a basket-centrifuge at a speed of 1200 rpm. The obtained starch cake is re-slurrified in 35 I of water and centrifuged a second time. The resulting starch cake is dried in a flash dryer at an inlet temperature of 160°C and an outlet temperature of 60° C

### C. Gelatinization

The modified granular starch cake is diluted in demineralized water in order to form a slurry at a concentration of about 8% calculated on dry substance. The resulting slurry has a relative density of 1 032 kg/l compared to water. The pH of the modified starch slurry is adjusted to 6.0. The slurry is the heated to 160°C by direct steam injection (Schlick Model 825). The temperature variation is not higher than ±1°C. The slurry is held in a holding column for a period of 4 minutes at a temperature of 160°C and a pressure of about 5 5 bar The pressure is then reduced to atmospheric by passing through a flash The slurry is then contained at 95°C in a hold tank.

### D. Spray-Drying

The slurry from Part C is carried out using a Niro FSD 4 spray-drying tower equipped with a 0.8 mm nozzle and fed at 10 l/hour. The inlet temperature is fixed at 300°C. and the outlet temperature of 120°C The obtained powder is a controlled release excipient with the following properties:

| Properties | |
|---|---|
| Moisture Content | 5 2% |
| Bulk Density | 103 g/l |
| Packed Density | 155 g/l |
| pH | 5 3 |
| Particle Size Peak Value | 70 µm |
| (Laser Particle Sizer-Sympatec) | |

Lubritab^{®} is a product sold by Penwest Pharmaceuticals Co (Cedar Rapids, lA, USA) Kollidon^{™} SR is a product produced by BASF (Germany) Encompress^{™} is a dicalcium phosphate dihydrate which can be purchased from Mendell (Patterson, NY). Tramadol hydrochloride can be obtained from Chemagis Ltd., 3 Hashlosha Street, P.O Box 9091, 61090, Tel Aviv, Israel Methods of synthesis and purification of tramadol are described in, for example, U S Patent Nos., 3,652,589, 5,414,129, 5,672,755, 5,874,620, 5,877,351, and 6,169,205.

### Manufacturing Procedare

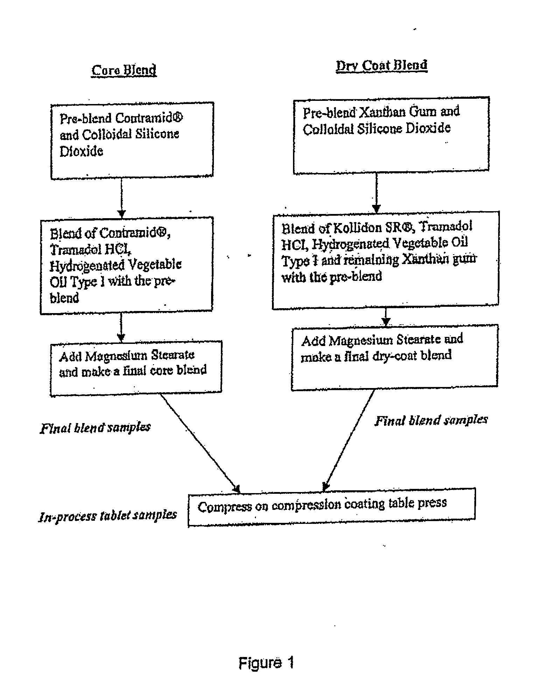

Tablets of the invention can be manufactured according to the process set out generally in the flow chart of Figure 1, and described In more detail below

**Weighing:** Raw materials are dispensed into clearly labeled containers

**Core Pre-Blend:** Blend a portion of the Contramid® and Colloidal Silicon Dioxide and pass through #30 mesh screen into a suitable container.

**Core Blend:** Place a portion of the Conframid® into a blender Pass Tramadol Hydrochloride through a #30 mesh screen and add to blender. Rinse container with a portion of Contramid® and add to blender Sieve Hydrogenated Vegetable Oil Type I through a #30 mesh screen and add to the blender Add the Core Pre-Blend into the blender. Add the remaining Contramid® into the blender, and blend all ingredients. Sieve the Magnesium Stearate through a #30 mesh screen and add blend with other ingredients Dispense blend in suitable container and Identify as Core Blend.

**Dry Coated Pre-Blend:** Blend a portion of the Xanthan Gum and all of the Colloidal Silicon Dioxide and pass through #30 mesh screen.

**Dry Coated Blend:** Place a portion of the Kollidon® SR into a blender Pass Tramadol Hydrochloride through Kason Separator with a #30 mesh screen into suitable container and add to blender. Rinse container with remaining xanthan gum and add to blender. Sieve Hydrogenated Vegetable Oil Type 1 through a #30 mesh screen and add to the blender. Place Dry Coated Pre-Blend and the remainder of the Kollidon® SR into the blender, and blend with all ingredients Sieve the magnesium stearate through a #30 mesh screen and blend with other ingredients. Dispense granulation in suitable container and identify as Dry Coated Blend

**Compression:** Use a Manesty Dry-Cota press to produce compression-coated tablets

### Example 3

Formulations A, B, and C, as shown in Table 1, were manufactured according to the process set out above

**Table 1: Recipes for Controlled Released Tramadol Formulations A, B and C.**

| **1) INGREDIENT** | **Formulation A** | | | **Formulation B** | | | **Formulation C** | |
|---|---|---|---|---|---|---|---|---|
| | | **%** | **mg/tablet** | | **%** | **mg/tablet** | **%** | **mg/tablet** |
| **Core** | | | | | | | | |
| Tramadol Hydrochloride | | 50 | 45 | | 50 | 90 | 63.25 | 151.8 |
| Contramid® | | 48.3 | 43.47 | | 48.3 | 86.94 | 35.05 | 84.1 |
| Hydrogenated Vegetable Oil | | 0.75 . | 0.675 | | 0.75 | 1.35 | 0.75 | 1.8 |
| Silica | | 0.2 | 0.18 | | 0.2 | 0.36 | 0.20 | 0.5 |
| Magnesium Stearate | | 0.75 | 0.675 | | 0.75 | 1.35 | 0.75 | 1.8 |
| Core Total Weight | | 100 | 90 | | 100 | 180 | 100 | 240 |

| **2) COAT** | | | | | | | | |
|---|---|---|---|---|---|---|---|---|
| Tramadol Hydrochloride | | 21.15 | 55 | | 30.56 | 110 | 30.56 | 148.5 |
| Silica | | 0.20 | 0.52 | | 0.20 | 0.72 | 0.20 | 1.0 |
| Kollidon SR® | | 51.42 | 133.7 | | 45.16 | 162.58 | 45.16 | 219 |
| Xanthan Gum | | 25.72 | 66.86 | | 22.58 | 81.3 | 22.58 | 109.5 |
| Hydrogenated Vegetable Oil | | 1.00 | 2.6 | | 1.00 | 3.6 | 1.00 | 4.9 |
| Magnesium Stearate | | 0.50 | 1.3 | | 0.50 | 1.8 | 0.50 | 2.4 |
| Coat Total Weight | | 100 | 260 | | 100.00 | 360 | 100 | 485 |

| **3) COATED TABLET** | | | | | | | | |
|---|---|---|---|---|---|---|---|---|
| Tramadol Hydrochloride | | 28.57 | 100 | | 37.04 | 200 | 41.38 | 300 |
| Contramid® | | 12.42 | 43.47 | | 16.10 | 86.94 | 11.60 | 84.1 |
| Hydrogenated Vegetable Oil | | 0.94 | 3.275 | | 0.92 | 4.95 | 0.92 | 6.7 |
| Silica | | 0.20 | 0.7 | | 0.20 | 1.08 | 0.20 | 1.5 |
| Magnesium Stearate | | 0.56 | 1.975 | | 0.58 | 3.15 | 0.58 | 4.2 |
| Kollidon SR® | | 38.20 | 133.7 | | 30.11 | 162.58 | 30.21 | 219 |
| Xanthan Gum | | 19.11 | 66.86 | | 15.06 | 81.3 | 15.10 | 109.5 |
| **Coated Tablet Total weight:** | | 100 | 350 | | 100 | 540 | 100 | 725 |

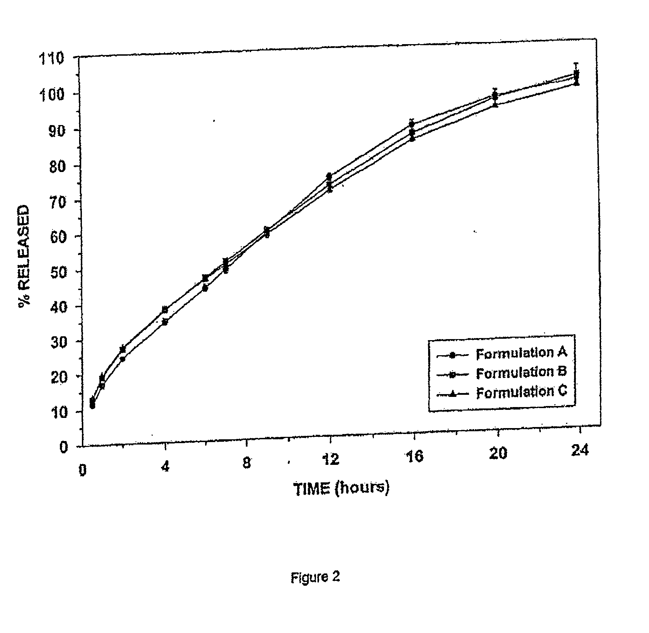

### Dissolution profiles of formulations A, B and C are shown in Figure 2.

### Tramadol Once Daily Formulation

The present invention relates to a controlled release tablet composition which provides analgesic effect within 2 hours of oral administration and lasts for at least 24 hours after administration

The 200 mg dose of the inventive controlled release composition surprisingly provides a rapid onset of analgesic effect within 2 hours after oral administration, and a mean tramadol plasma concentration between 100 ng/mL and 200 ng/mL for at least 24 hours after a single dose.

Furthermore, at steady-state, the mean tramadol plasma concentration remains between 100 ng/mL and 350 ng/mL. The inventive controlled release compositions have surprisingly been shown to provide full clinical effect for at least 24 hours after oral administration

### Bioavailability Studies

An object of the present invention is to provide flexible dosing options for patients with different analgesic requirements with a once-a-day formulation, which upon ingestion, would maintain the desired early onset of action but achieve mean tramadol plasma concentrations of at least 45 ng/mL between 2 and 24 hours or at least 100 ng/mL between 2 and 22 hours or at least 150 ng/mL between 2 and 24 hours after one dose depending upon the dosage administered

### EXAMPLE 4

### (i) Dose Proportionality - Single Dose

A bioavailability study was conducted to assess the dose-proportionality between three dosage strengths (100 mg, 200 mg and 300 mg). This study was conducted with a suitable washout period between each administration The doses were taken by 27 healthy human volunteers under fasting conditions

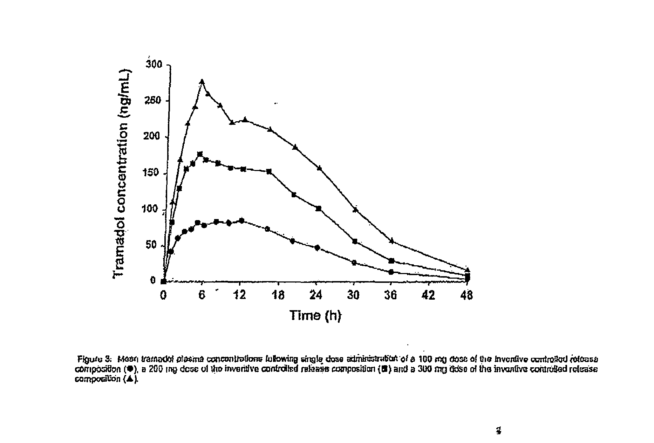

Figure 3 depicts the mean plasma concentration time-profiles of tramadol obtained in the subjects after the administration of the inventive controlled release composition (dosed at 100 mg, 200 mg and 300 mg of tramadol HCl) The data used to create Figure 3 is included in Table 2.

**Table 2 Mean (±SD) Tramadol Plasma Concentrations (ng/mL)**

| Time | 100 mg dose of the inventive controlled release composition | 200 mg dose of the inventive controlled release composition | 300 mg dose of the inventive controlled release composition |
|---|---|---|---|
| 0 | | 0 | 0 |
| 1 | 418 ± 14.1 | 82.5 ± 24.1 | 110.2 ± 36 7 |
| 2 | 60.0 ± 146 | 129.2 ± 25.7 | 168.6 ± 52.1 |
| 3 | 69 2 ± 20.2 | 1565 ± 37.0 | 218 1 ± 82 3 |
| 4 | 72.5 ± 21.8 | 164.0 ± 44.9 | 242.0 ± 96.2 |
| 5 | 817 ± 24.2 | 177.2 ± 61.8 | 277.1 ± 153.8 |
| 6 | 77.9 ± 24 7 | 169.2 ± 58 1 | 260.3 ± 134.8 |
| 8 | 83 0 ± 25 6 | 164.1 ± 52 7 | 243.6 ± 127.1 |
| 10 | 81.0 ± 24 7 | 157.8 ± 57 8 | 219.8 ± 101.6 |
| 12 | 84.4 ± 25 3 | 156.4 ± 55 9 | 223.4 ± 85.1 |
| 16 | 73.0 ± 24 1 | 152.8 ± 42.0 | 209.9 ± 70.2 |
| 20 | 56.4 ± 19 4 | 121.0 ± 34.4 | 185.7 ± 62.7 |
| 24 | 47.2 ± 20.9 | 101.6 ± 38.2 | 157.0 ± 60.4 |
| 30 | 26.8 ± 15.0 | 56-4 ± 28.3 | 99.9 ± 50.3 |
| 36 | 13.2 ± 9.4 | 291 ± 18.7 | 55.9 ± 37.9 |
| 48 | 3.7 ± 3.5 | 8.5 ± 6.7 | 15.7 ± 13.1 |

The results from this study indicated that the 100 mg, 200 mg and 300 mg formulations of the inventive controlled release composition are dose proportional With respect to the rate and extent of absorption of tramadol and the rate and extent of formation of O-desmethyltramadol.

Bioavailability studies were conducted in order to characterize the pharmacokinetic properties of the inventive controlled release composition and to demonstrate similar exposure of the drug and/or its active metabolite when compared to a reference product

### Example 5

### (ii) Comparison to a Twice Daily Formulation-Single Dose

The 2 x 200 mg dosage of the inventive controlled release composition was compared to the twice daily formulation, Topalgic® LP (200 mg) tablets manufactured by Laboratoires Hoechst Houdé in a comparative bioavailability study after administration under fasting conditions in 24 healthy human volunteers.

The pharmacokinetic results from the inventive controlled release composition were compared to those obtained following twice daily administration (at 12-hour intervals) of the reference formulation in order to assess bioequivalence between the test and the reference product Based on calculation of the 90% confidence interval of the test versus reference ratios of geometric means, the extent of exposure (determined by assessment of AUC₀₋ₜ and AUC_{0-∞} of tramadol, following dose normalization) was within the conventional bioequivalence interval of 80-125% for the log-transformed parameters Thus the inventive controlled release composition and the twice daily formulation were found to be bioequivalent in terms of the overall exposure to tramadol Results for tramadol AUC_{0-∞} are presented in Table 3.

**Table 3 Comparison of AUC_{0-∞} (Single-dose versus twice-daily formulation)**

| Treatment | Arithmetic Mean ± SD (ng·h/mL) | Geometric Mean Ratio (90% Confidence Interval) (90% Confidence Interval) |
|---|---|---|
| 2 x 200 mg dose of the inventive controlled release composition | (ng·h/mL) 9332 ± 3767 | 103(98-109) |
| 1 x 200 mg Topalgic® LP BID | 8897 ± 3124 | |

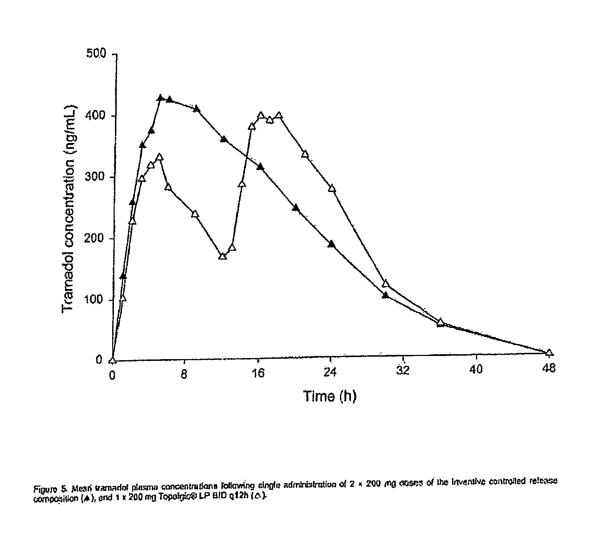

Figure 5 depicts the arithmetic mean plasma concentration time-course profiles of tramadol obtained after the administration of the inventive controlled release composition once a day and of the reference product in one day at 12-hour intervals in the 24 healthy volunteers. The data used to create Figure 5 is included in Table 4

**Table 4 Mean Tramadol Plasma Concentrations (ng/mL)**

| Test formulation | | Reference formulation | |
|---|---|---|---|
| Time | Conc. 2x200 mg dose of the inventive composition | Time | Conc. 200 mg BID |
| | 0 | 0 | 0 |
| 1 | 138.49 ± 58.62 | 1 | 101.93 ± 43.72 |
| 2 | 257.56 ± 81.20 | 2 | 226.89 ± 72.90 |
| 3 | 35021 ± 165.42 | 3 | 295.35 ± 99.46 |
| 4 | 373.93 ± 124.33 | 4 | 31822 ± 91.27 |
| 5 | 427.66 ± 166.90 | 5 | 330.88 ± 98.68 |
| 6 | 424.72 ± 176.20 | 6 | 281.67 ± 85.95 |
| 9 | 408.61 ± 196.28 | 9 | 236.39 ± 87.89 |
| 12 | 357.88 ± 162.48 | 12 | 16741 ± 66.49 |
| 16 | 312.70 ± 153.34 | 13 | 181.96 ± 70.51 |
| 20 | 243.94 ± 117.93 | 14 | 284.67 ± 126.76 |
| 24 | 184.96 ± 102.90 | 15 | 378.82 ± 136.23 |
| 30 | 99.78 ± 61.60 | 16 | 396.87 ± 146.66 |
| 36 | 51.01 ± 43.33 | 17 | 388.83 ± 142.32 |
| 48 | 0 | 18 | 396.38 ± 140.65 |
| | | 21 | 331.81 ± 121.52 |
| | | 24 | 275.00 ± 110.61 |
| | | 30 | 118.69 ± 64.92 |
| | | 36 | 54.04 ± 39.07 |
| | | 48 | 0 |

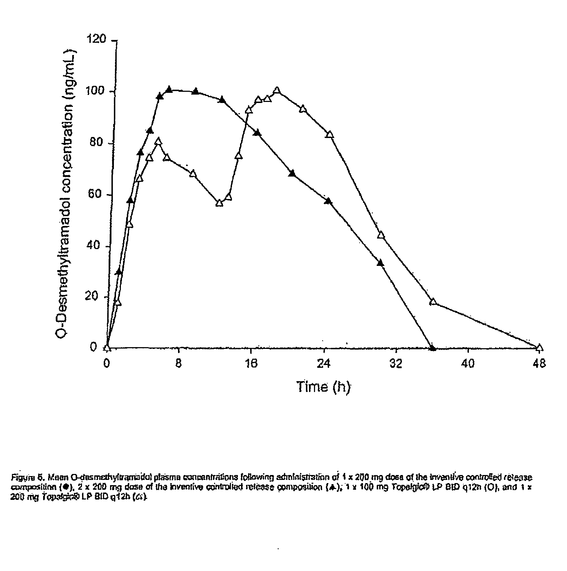

Figure 6 depicts the arithmetic mean plasma concentration time-course profiles of O-desmethyltramadol obtained after the administration of the inventive controlled release composition once-a-day and of the reference product in one day at 12-hour intervals in the 24 healthy volunteers. The data used to create Figure 6 is included in Table 5

**Table 5 Mean(±SD) O-desmethyltramadol Plasma Concentrations (ng/mL)**

| Test formulation | | Reference formulation | |
|---|---|---|---|
| Time | Conc. 2x200 mg dose of the inventive composition | Time | Conc. 200 mg BID |
| 0 | 0 | 0 | 0 |
| 1 | 29.82 ± 17.0 | 1 | 17.7 ± 14.6 |
| 2 | 57.8 ± 17.0 | 2 | 48.3 ± 17.5 |
| 3 | 76.3 ± 31.6 | 3 | 65.2 ± 25.9 |
| 4 | 84.9 ± 309 | 4 | 74.3 ± 26.2 |
| 5 | 98.0 ± 41.4 | 5 | 80.64 ± 29.2 |
| 6 | 100.6 ± 41.7 | 6 | 74.3 ± 26.1 |
| 9 | 99.9 ± 41.7 | 9 | 68.1 ± 24.6 |
| 12 | 96.52 ± 38.8 | 12 | 56.6 ± 22.1 |
| 16 | 83.9 ± 32.6 | 13 | 59.1 ± 23.8 |
| 20 | 68.2 ± 28.8 | 14 | 75.1 ± 32.6 |
| 24 | 576 ± 28.0 | 15 | 92.6 ± 38.0 |
| 30 | 332 ± 20.0 | 16 | 96.7 ± 37.0 |
| 36 | 0 | 17 | 97.0 ± 34.5 |
| 48 | 0 | 18 | 100.4 ± 33.6 |
| | | 21 | 93.0 ± 32.4 |
| | | 24 | 83.3 ± 37.8 |
| | | 30 | 44.4 ± 21.6 |
| | | 36 | 18.1 ± 16.8 |
| | | 48 | 0 |

### Example 6

### (iii) Comparison to a Twice Daily Formulation - Steady State

The 200 mg dosage of the inventive controlled release composition was compared to the twice daily formulation, Topaigic® LP (100 mg) tablets, manufactured by Laboratoires Hoechst Houdé, in a comparative bioavailability study after multiple administration under fasting conditions in 26 healthy human volunteers.

The results from this study indicated that the inventive controlled release composition is equivalent to the reference product with respect to the rate and extent of absorption of tramadol and the rate and extent of formation of O-desmethyltramadol. The comparative bioavailability of the two products was assessed on the basis of the confidence interval for the primary variable AUCₛₛ for tramadol and O-desmethyltramadol in relation to the conventional bioequivalence range of 80% to 125%. Results for tramadol AUCₛₛ are presented in Table 6

**Table 6 Comparison of AUCₛₛ (Once-a-day versus twice-daily formulation)**

| Treatment | Arithmetic Mean ± SD (ng h/mL) | Geometric Mean Ratio (90% Confidence Interval) |
|---|---|---|
| 200 mg dose of the inventive controlled release composition | 5185±1460 | 92.4 (87 5-975) |
| Topalgic® LP 100 mg BID | 5538 ± 1214 | |

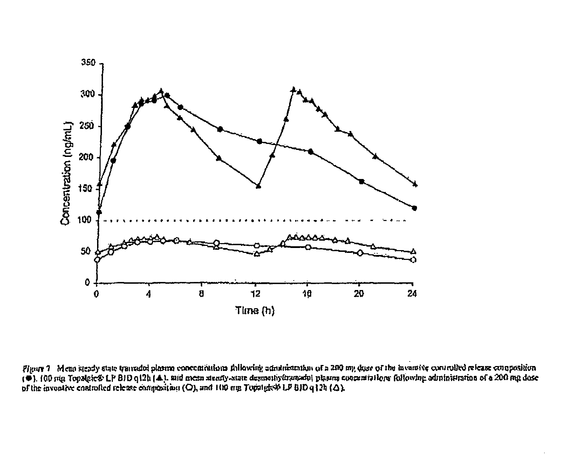

Figure 7 depicts the arithmetic mean plasma concentration time-course profiles of tramadol and O-desmethyltramadol following administration of a 200 mg dose of the inventive controlled release composition once a day and of the reference product (Topalgio® LP 100 mg BID) in one day at 12 hour intervals. The data used to create Figure 7 is included in Table 7.

**Table 7 Mean (±SD) Tramadol and O-desmethyltramadol Plasma Concentrations mg/mL)**

| Test formulation (200 mg dose of the inventive controlled release composition) | | | Reference formulation (100 mg BID) | | |
|---|---|---|---|---|---|
| Time | Tramadol | Metabolite | Time | Tramadol | Metabolite |
| 0 | 113.3 ± 48 8 | 37.6 ± 9.0 | 0 | 1578 ± 48.8 | 49.1 ± 107 |
| 1 | 195.4 ± 58.4 | 499 ± 13.9 | 1 | 2202 ± 611 | 581 ± 129 |
| 2 | 2 249.5 ± 61 0 | 58.9 ± 144 | 2 | 2 2516 ± 60.9 | 63.1 ± 146 |
| 3 | 2850 ± 66 0 | 65.4 ± 163 | 2.5 | 282.7 ± 65.3 | 680 ± 14 7 |
| 4 | 290.6 ± 65 5 | 66.2 ± 16.0 | 3 | 290.8 ± 59 7 | 69.4 ± 15.6 |
| 5 | 298 9 ± 81 1 | 67 3 ± 16.7 | 3.5 | 290.9 ± 70 6 | 69 6 ± 15.7 |
| 6 | 280.0 ± 70 7 | 67.7 ± 17 5 | 4 | 4 297.3 ± 71 3 | 71.3 ± 15 3 |
| 9 | 244 9 ± 58.4 | 63 9 ± 16.8 | 4.5 | 305 2 ± 75.2 | 72 8 ± 15 6 |
| 12 | 226.0 ± 70.2 | 59 8 ± 17 2 | 5 | 281 8 ± 65.5 | 69.1 ± 15 7 |
| 16 | 209 4 ± 73.4 | 57.3 ± 14.8 | 6 | 262.8 ± 55.5 | 67 4 ± 17.3 |
| 20 | 161.5 ± 68 9 | 47.9 ± 12.1 | 7 | 243 9 ± 60.2 | 64.9 ± 15.2 |
| 24 | 118.9 ± 59 1 | 37.1 ± 8 9 | 9 | 198.0 ± 54 4 | 57.0 ± 12 8 |
| | | | 12 | 154 6 ± 47.8 | 46 2 ± 10.5 |
| | | | 13 | 203.5 ± 55.4 | 53.2 ± 12 8 |
| | | | 14 | 260.7 ± 54.2 | 63.7 ± 15 0 |
| | | | 14.5 | 307.2 ± 59 9 | 72.2 ± 16 5 |
| | | | 15 | 303 7 ± 60.5 | 73 2 ± 17.1 |
| | | | 15.5 | 290.7 ± 54 3 | 71.3 ± 16 8 |
| | | | 16 | 28 9 ± 54.6 | 72 1 ± 15.6 |
| | | | 16.5 | 276.4 ± 53.2 | 72 1 ± 16.8 |
| | | | 17 | 267.6 ± 55 2 | 71.0 ± 16 8 |
| | | | 18 | 244 6 ± 58 4 | 68.2 ± 15.0 |
| | | | 19 | 237,1 ± 59 4 | 66.4 ± 14 8 |
| | | | 21 | 201 5 ± 52.7 | 57 9 ± 12.0 |
| | | | 24 | 156.9 ± 49.9 | 49.6 ± 10.1 |

### Efficacy of Inventive Composition Compared to Twice-Daily Formulation

The inventive controlled release composition has surprisingly been shown to result in a clinically favourable safety profile when compared with a twice daily tramadol formulation Furthermore, the inventive controlled release composition has been shown to provide analgesic efficacy over the 24-hour dosing interval which is at least as good as a twice daily formulation

A multicentre clinical trial was performed in Europe with the objective of comparing the efficacy of the present invention to commercially available twice daily tramadol HCl sustained release (Tramadol BID) tablets (manufactured by Grünenthal GmbH) in 431 patients treated for pain due to osteoarthritis (OA) of the knee for 12 weeks in addition, the clinical trial was conducted to compare the safety and clinical benefit of the present invention to the Tramadol BID tablets in patients treated for pain due to OA of the knee for 12 weeks Osteoarthritis of the knee is widely used as a chronic pain model for studying the efficacy of pain medications and was therefore considered to be appropriate for the study

This study demonstrated that the inventive controlled release composition provides sustained analgesic efficacy over the entire 24-hour dosing interval and a clinically favourable safety profile. This once daily dosing regimen and favourable safety profile could improve convenience and regimen compliance in clinical settings.

This multi-centre study consisted of 3 phases: Baseline, Titration and Maintenance

At the beginning of the baseline phase, all pain medication and prohibited medications were discontinued for at least 5 half-lives prior to randomisation in order to obtain unbiased baseline values for the efficacy variables and to ensure that patients had sufficlently severe OA (moderate to moderately severe) On the final day of the Baseline Phase, eligible patients were randomly assigned to a treatment group (inventive formulation or tramadol BID). Patients then entered the Titration Phase (4 to 12 days), during which the tramadol daily dose was increased or decreased by increments of 100 mg every 2-3 days based upon efficacy and tolerability until an optimum dose was established (range 100-400 mg) Patients were then treated at the fixed optimum dose for the duration of the Maintenance Phase (12 weeks). Patients were discontinued if they required a change in dose during the Maintenance Phase The efficacy and safety of the study medication were evaluated during study visits which occurred at the end of titration and at Weeks 3 and 6 and the final visit (either Week 12 or at the time of premature discontinuation) during the Maintenance Phase

The selection of dose range (100 mg to 400 mg daily) was based upon the approved dose range for the various marketed formulations of tramadol (Topalgic® in Vidal 2001: Le dictionnaire 77e edition Paris Editions du Vidal, 2001. pp 2058-2060; Ultram® in Physicians' Desk Reference, 2002) For patients randomised to the tramadol BID group, placebo was used at the 100 mg dose level to maintain the study blind (50 mg BID tablets not being available) Therefore, patients in this group took their first active medication at the 200 mg dose level which is consistent with the manufacturer's usual dosing recommendations. Doses of the inventive controlled release composition were composed of 100-mg and 200-mg tablets while tramadol BID doses were composed of 100-mg, 150-mg and 200-mg tablets.

The primary efficacy measure was the rating of pain as assessed by the WOMAC pain sub-scale. Secondary efficacy measures included the WOMAC stiffness and physical function sub-scale scores and WOMAC global score, a Likert-scale rating of pain at the end of the dosing interval, Visual Analog Scale (VAS) pain ratings over the 24 hours prior to each visit and patient and investigator global ratings of pain.

The WOMAC index is a statistically validated, 24-item questionnaire divided into 3 sub-scales (Pain; 5 questions, Stiffness: 2 questions, and Physical-function: 17 questions) (See Bellamy, N et al "Relationship between severity and clinical importance of symptoms in osteoarthritis." Clin Rheumatol 1991; 10·138-143; Theiler, R et al "Superior responsiveness of the pain and function sections of the Western Ontario and McMaster Universities Osteoarthritis Index (WOMAC) as compared to the Lequesne-Algofunctional Index in patients with osteoarthritis of the lower extremities." Osteoarthritis Cartilage 1999; 7: 515-519). The WOMAC Global score consists of the total of the 3 sub-scales. Each of the questions is answered using a 100-mm VAS. The WOMAC questionnaire was administered to patients at each study visit (baseline, end of titration and Week 3, 6 and 12 or premature discontinuation). Composite scores for each sub-scale and the global score were reported separately

The Likert-scale rating of pain at the end of dosing interval required that patients rate their pain each morning using a 4-point Likert-scale (none = 0, barely noticeable =1, mild = 2, moderate = 3, severe = 4) Because of the difference in dosing intervals for the two medications, patients taking the inventive controlled release composition rated their pain 24 hours after their previous dose and tramadol BID patients rated their pain 12 hours after their previous dose.

The VAS ratings of pain over 24 hours were administered at each visit Patients rated the current, average, least and worst pain experienced over the previous 24 hours using a 100-mm VAS scale.

Global ratings of the effect of the medication on pain control were completed at each visit after baseline by both the patients and the investigators Responses were given using a 4-point Likert scale (very effective, effective, somewhat effective, ineffective).

Information on clinical laboratory tests, physical exams, adverse events and concomitant medications was used to evaluate safety during the trial

Analysis of the data utilised 3 populations: the Intent-to-treat (ITT) Population, the Per-Protocol (PP) Population and the Safety Population. Efficacy analysis was performed using the ITT and PP populations with the PP population being the primary analysis population The ITT Population was defined as ail randomized patients who received study medication and had at least one post baseline assessment of any functional scale. The PP Population was defined as a subgroup of the ITT population who completed the study, for whom no major protocol deviations either before or during the trial were observed and for whom a pain rating was recorded at the end of the Maintenance Phase. Early dropouts due to adverse events or lack of efficacy were included in the PP population provided they took study medication for at least two weeks during the Maintenance Phase and had efficacy assessments within 2 days after the end of study medication The Safety population was defined as all patients who received at least 1 dose of randomised study medication

A one-sided 97.5% confidence Interval (CI) for the group difference "test treatment minus reference treatment" was derived from a 1-way ANCOVA and non-inferiority of the test treatment was confirmed if the lower limit of the confidence interval was greater than or equal to minus delta (15%). The WOMAC pain score (primary efficacy variable) reported as the percentage of change between Baseline and Day 84, was calculated as follows- ((Baseline - Day 84)/Baseline) x 100 In the case of premature discontinuation, the last assessment was used to calculate the percentage of change. Similarly, treatment differences, with respect to changes between baseline and Week 12 based on the WOMAC Global score and the stiffness and physical function sub-scales were assessed based upon the ANCOVA model but the results were interpreted at a descriptive level only

A total of 431 patients were randomized The Safety population and the ITT populations included the 430 patients who took randomised study medication (one randomised patient never took any study medication). For inclusion in the ITT population, patients were also required to have at least 1 post-baseline assessment (Table 8).

**Table 8: Populations**

| Population | | inventive controlled release composition | Tramadol BID |
|---|---|---|---|
| Randomised | N=431 | 215 | 216* |
| Safety Population • Received at least 1 dose of study N=430 medication | | 215 | 215 |
| ITT Population • Received at least 1 dose of N=430 medication and at least 1 post baseline assessment | | 215 | 215 |
| PP Population • Completed the study or received study medication for at least 2 weeks during the Maintenance phase 2 N=314 • Had efficacy assessments within days of last dose • Had no major protocol violations | | 161 | 153 |

| | | | |
|---|---|---|---|
| Table 8: One patient who was randomised never look any medication | | | |

Sixteen percent (16%) of the patients in the ITT population discontinued from the study prior to Week 12. The reasons for premature discontinuation in the ITT population are listed in Table 9.

**Table 9: Frequency and reason for premature discontinuation ^{a}**

| Reason for Early Discontinuation | Inventive controlled release composition N=215 | Tramadol BID N=215 |
|---|---|---|
| Adverse events | 19 | 22 |
| Death⁸ | 1 | - |
| Treatment faillure | 2 | 2 |
| Protocol violations | 3 | 4 |
| Patient request | 8 | 9 |
| Total | 33 | 37 |

| | | |
|---|---|---|
| Table 9: ^{a} ITT Population; 67-year old female: ischaemic stroke, considered not related to study medication ' | | |

The PP population consisted of the 314 patients who remained, after the exclusion of major protocol violators from the ITT population (Table 8). Fifty-four (54) patients taking the inventive controlled release composition and 62 tramadol BID patients were excluded from the PP population. The most frequent reasons for exclusion were: no WOMAC pain score at baseline or final visit, whether that was Week 12 or an early discontinuation (41 in each treatment group); intake of prohibited prior or concomitant medications (inventive composition: 7; tramadol BID- 15), major violation of eligibility criteria (inventive composition: 7; tramadol BID: 5), incorrect intake of study medication (inventive composition 2, tramadol BID. 5) and treatment duration out of the allowed window (12 weeks ±10 days of the scheduled treatment duration)( inventive composition 4, tramadol BID: 5) Both treatment groups in the PP population were similar with no statistical differences regarding diagnosis, gender distribution, age, mean BMI and mean pain at baseline (WOMAC pain sub-scale score) (Table 10).

**Table 10: Baseline characteristics ^{a}**

| | | Inventive controlled release composition N=161 | Tramadol BID N=153 |
|---|---|---|---|
| Gander | | | |
| Male | N(%) | 31(19.3%) | 24(15.7%) |
| Female | N(%) | 130(80.7%) | 129(84.3%) |
| | | | |
| Age (yrs) | | | |
| Mean±SD | | 60.8 ± 9.3 | 60.3 ± 9.3 |
| Range | (min, max) | 40-75 | 39-75 |
| <65 yrs | N(%) | 96 (59.6%) | 93 (60.8%) |
| >65 yrs | N(%) | 65 (40.4%) | 60 (39.2%) |
| | | | |
| BMI (kg/m²) | | 29.2 ± 3.5 | 28.6 ± 3.8 |
| Mean ± SD | | | |
| | | | |
| WOMAC Pain Score at Baselina(mm) | | | |
| Mean ± SD | | 285 mm ± 71.0 | 292 mm ± 70.5 |

| | | | |
|---|---|---|---|
| Table 10^{-a} PP population | | | |

Use of analgesics (other than the study medication), sedative hypnotics, anesthetics and muscle relaxants were not permitted during the trial These medications had to be washed out for 5 half-lives prior to randomisation.

Ninety percent (90%) of the patients in the group taking the inventive controlled release composition and 92% of those in the Tramadol BID group were treated with one or more medications prior to the start of the study The most commonly reported prior medications were taken for pain or cardiac disorders. Both treatment groups were similar with regard to the use of analgesics prior to the start of the study (salicylate derivatives and related substances inventive composition 37%; tramadol BID 38%; opioids inventive composition 13%; tramadol BID 11%)(Table 11)

**Table 11 Prior and Concomitant Medications^{a}**

| | Inventive Controlled Release Composition N = 215 | | Tramadol BID N = 215 | | Overall N = 430 | |
|---|---|---|---|---|---|---|
| Prior Medications⁶ | 193 | (89.8%) | 197 | (91.6%) | 390 | (90.7%) |
| Salicylate derivatives and related substances | 79 | (36.7%) | 82 | (38 1%) | 161 | (37.4%) |
| Other opioids | 28 | (13.0%) | 24 | (11.2%) | 52 | (12.1%) |
| Prior and Concomitant Medications | | | | | | |
| ACE-inhibitors | 64 | (298%) | 71 | (33.0%) | 135 | (31.4%) |
| Beta blocking agents | 40 | (18.6%) | 26 | (121%) | 66 | (153%) |
| Organic nitrates | 31 | (144%) | 26 | (12.1%) | 57 | (133%) |
| Dihydropyridine derivatives | 16 | (7.4%) | 27 | (12.6%) | 43 | (10.0%) |

| | | | | | | |
|---|---|---|---|---|---|---|
| Table 11: Medications taken prior and concomitant to study entry by more than 10% of patients; ^{a} ITT Population; ^{b} prior medications which were discontinued 5 half-lives prior to randomisation; ° prior medications which patients continued to take during the study | | | | | | |

Patients were permitted to continue treatment with all other medications with the exception of monoamine oxidase inhibitors, tricyclic antidepressants, neuroleptics, selective serotonin reuptake Inhibitors or other drugs that reduce seizure threshold Both groups were similar with regard to use of ACE inhibitors (inventive composition 30%; tramadol BID 33%) and organic nitrates (inventive composition 14%; tramadol BID 12%) However, more patients took dihydropyridine derivatives in the tramadol BID group (inventive composition 7%; tramadol BID 13%) whereas more patients took beta-blocking agents (inventive composition 19%; tramadol BID 12%) in the inventive composition group (Table 11)

Treatment for adverse events was allowed, as necessary, in order to permit patients to continue in the study The adverse events most often requiring treatment with concomitant medications were constipation (18% of patients) and nausea (6% of patients)

The median optimal daily dose received was 200 mg in both treatment groups Only 34% of the patients taking the inventive composition and 37% of the tramadol BID patients required doses of tramadol higher than 200 mg (Table 11) Compliance was within the range defined as 'acceptable' (80% to 120%) for all patients but one (71%) The mean exposure to study medication was similar between the two groups (82 days; range 3-121 days) with the majority of patients (79%) being dosed for 84 days or more

**Table 12: Optimum daily doses ^{a,b}**

| Optimum dose | Inventive Controlled Release Composition (N=161) | Tramadol BID (N=153) |
|---|---|---|
| | | |
| 100 mg | 15.5% | 13.7%^{c} |
| 200 mg | 50.9% | 49.0% |
| 300 mg | 25.5% | 26.1% |
| 400 mg | 8.1% | 11.1% |
| | | |
| Median dose | 200 mg | 200 mg |

| | | |
|---|---|---|
| Table 12 ^{a} Percentage of patients per dose; ^{b}PP Population; ^{c} As the manufacturers usual dosing recommendations Indicate that dosing should start at 200 mg, these patients received placebo at that dose level. | | |

Both the primary endpoint (percent change in WOMAC pain subscale) and all secondary efficacy endpoints were successfully achieved (VAS pain ratings over the 24 hours prior to visits; Likert-scale rating of pain at the end of the dosing interval; remaining WOMAC scores stiffness, physical function and global; and patient and investigator overall ratings of pain)

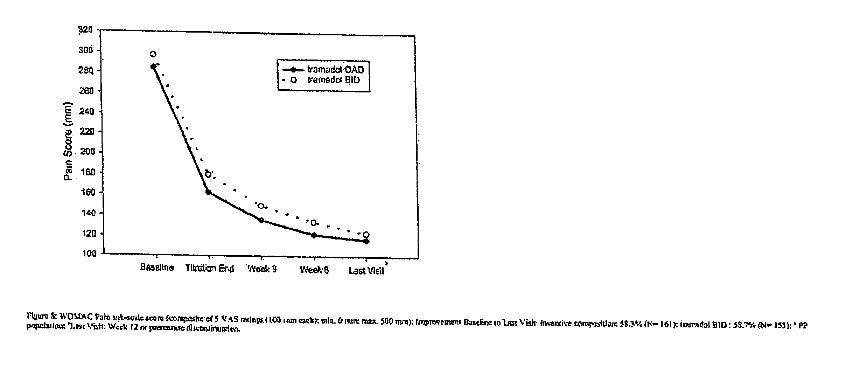

An immediate and significant improvement in the mean WOMAC pain score was seen during the Titration Phase for both treatments (inventive composition 43%; tramadol BID: 40%). Furthermore, improvement continued throughout the Maintenance Phase when patients were receiving their fixed optimum dose (inventive composition: 58%; tramadol BID 59%) (See Figure 8).

In order to assess the non-inferiority of the inventive composition compared to tramadol BID, a 2-way ANCOVA was performed on the percentage change in the WOMAC pain sub-scale score from baseline to the last visit while adjusting for the baseline score.

The percent improvement in WOMAC pain score from baseline to Week 12 was similar in both groups Non-inferiority of the inventive composition versus tramadol BID with respect to the WOMAC pain sub-scale score was demonstrated More precisely, the 95% Cl for the difference in improvement between treatments, estimated as (-7 67; 3.82), demonstrated the non-inferiority of the inventive composition compared to tramadol BID since its lower bound, corresponding to the lower limit of the 97.6% Cl, was greater than -15% (See Figure 8 and Table 13)

**Table 13 Pain as Assessed by the WOMAC Scale^{a,b}**

| Pain Score | Inventive Controlled Release Composition N=161 | Tramadol BID N=153 |
|---|---|---|
| | | |
| Baseline: | | |
| Mean ± SD | 284.9 ± 71.0 mm | 297.4 ± 66.9 mm |
| | | |
| End of Titration: (Visit 2) | | |
| Mean ± SD | 162.3 ± 93.8 mm | 180.0±99.2mm |
| | | |
| Last visit | | |
| Mean ± SD | 116.9 ± 88.2 mm | 123.2 ± 89.5 mm |
| | | |
| Improvement during Titration | | |
| Mean change (%) baseline to Visit 2 (95% Cl) | 426% (37 9-47.2) | 39 6% (34.9 - 44 3) |
| Total improvement | 58 3% | 58.7% |
| Mean change (%) baseline to Last Visit (95% Cl) | (53.7-629) | (54.4-63.0) |

| | | |
|---|---|---|
| Table 13: ^{a}WOMAC Pain sub-scale score(composite of 5 VAS ratings: min 0 mm; Max 500 mm); Last Visit Week 12 or premature discontinuation; ^{b}PP Population | | |

The comparison of VAS pain ratings over a 24-hour period showed similar significant improvement throughout the study (Table 14)

**Table 14: VAS pain ratings of pain over 24 hours Improvement(%) baseline to Last Visit ^{a,b}**

| | Inventive Controlled Release Composition N=161 | Tramadol BID N=153 |
|---|---|---|
| | | |
| Current Pain Change (%) baseline to Last Visit Mean ± SD | 35 1% ± 23.8 | 34.6%±235 |
| | | |
| Least Pain Change (%) baseline to Last Visit Mean ± SD | 21.6%±213 | 23.5%±21.2 |
| Worst Pain Change (%) baseline to Last Visit Mean ± SD | 38.6% ± 127.9 | 39.2% ± 26 3 |
| | | |
| Average Pain Change (%) baseline to Last Visit Mean ± SD | 30.1%±220 | 307%±197 |

| | | |
|---|---|---|
| Table 14. VAS pain rating (0-100 mm) over the 24 hours prior to each study visit; ^{a} last Visit: Week 12 or premature discontinuation; ^{b}PP Population | | |

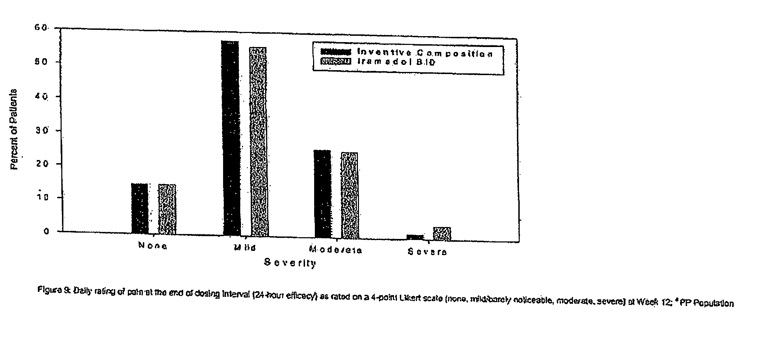

Both the inventive composition taken once every 24 hours and tramadol BID taken every 12 hours demonstrated similar efficacy at the end of the respective dosing intervals. In fact, in daily ratings, 73% of all patients indicated that their pain was mild/barely noticeable or absent at the end of the dosing interval (inventive composition 72 3%; tramadol BID 71 3%) (Figure 9)

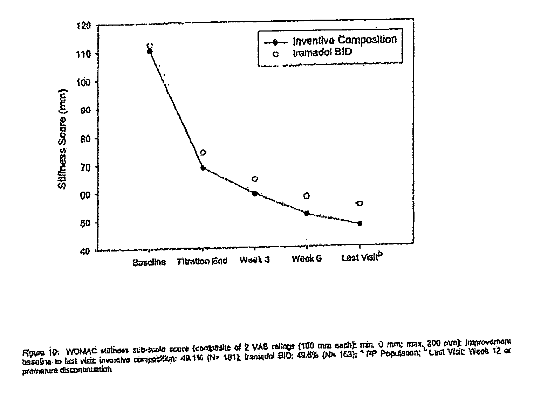

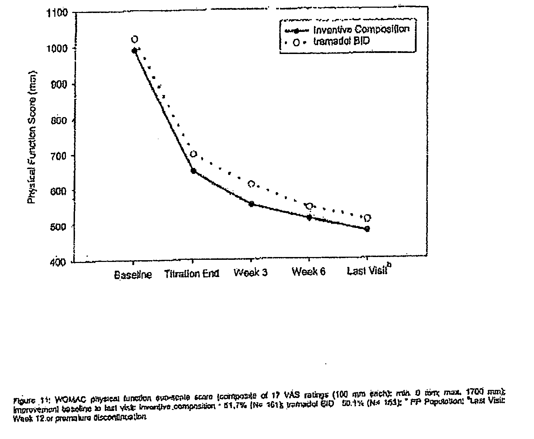

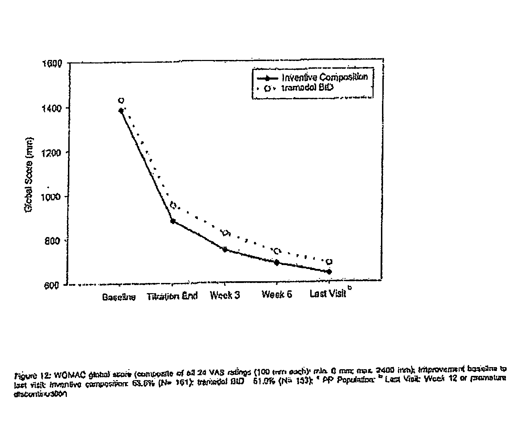

The WOMAC secondary efficacy analyses (stiffness, physical function and global) showed a pattern of efficacy similar to that of the WOMAC pain sub-scale in both groups with no significant difference between them. The trend for a marked decrease in pain during the titration phase with a continued decrease throughout the Maintenance Phase was sustained for all three of these secondary efficacy measures (WOMAC stiffness, physical function and global scores) (See Figures 10, 11 and 12).

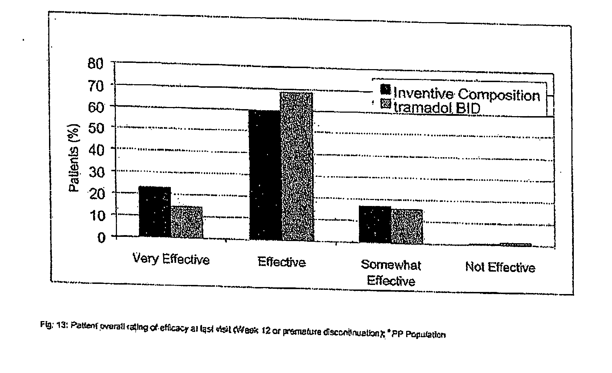

The majority of patients In both groups (83%) gave an overall rating of the study medication as effective or very effective (inventive composition 82 6%; tramadol BID 83.0%). No statistical differences were noted between the treatment groups (Figure 13).

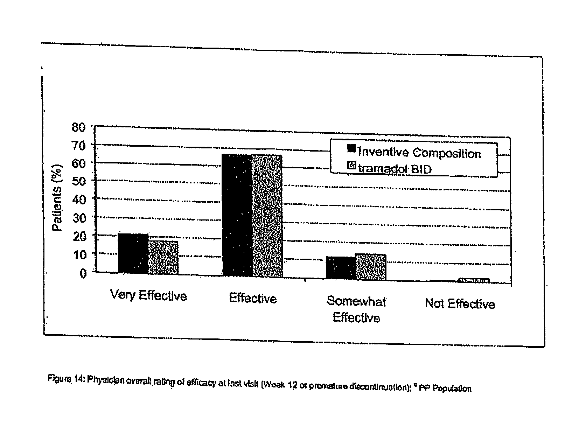

The majority of investigators (86%) also rated the overall efficacy of analgesia in both groups as very effective or effective (Figure 14)

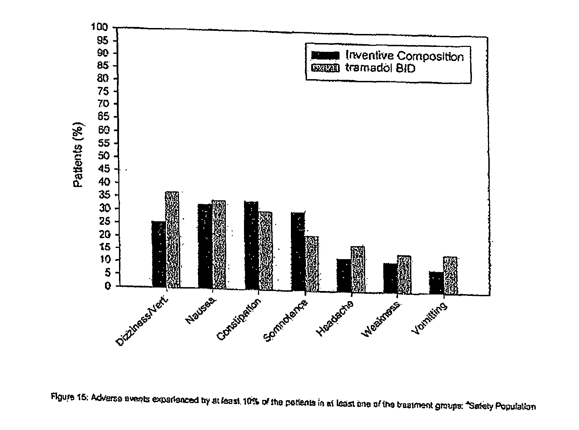

Safety data are reported for the ITT population (N=215 in each treatment group) A similar number of patients in each group reported at least 1 adverse event (inventive composition 81%; tramadol BID 79% p=0.6283). The most common AEs were gastrointestinal (GI) and central nervous system (CNS) related A greater number of tramadol BID patients reported dizziness/vertigo (inventive composition 26%; tramadol BID 37% p=0 0165), vomiting (inventive composition 8%; tramadol BID 14% p= 0 0677) and headache (inventive composition 13%; tramadol BID 18% p=0 1779), while more patients taking the inventive composition reported somnolence (inventive composition 30%; tramadol BID: 21% p=00470) Furthermore, the episodes of vomiting or dizziness/vertigo reported by the patients in the tramadol BID group were more severe than those reported in the group taking the inventive composition (Vomiting inventive composition 6% severe vs tramadol BID 23% severe; dizziness/vertigo inventive composition 2% severe vs tramadol BID 13% severe) Most of these adverse events were rated as mild or moderate (inventive composition 67%, tramadol BID: 64%) (See Table 16, Figure 15).

The mean time of onset of the most common AEs and their median duration was similar in both groups (median onset between 3 and 13 days and duration between 2 and 18 days depending upon the AE) (Table 15)

**Table 15: Time to onset (number of days) of most common adverse evente^{a,b}**

| | Inventive Controlled Release Composition N=215 | | | | Tramadol BID N = 215 | | | |
|---|---|---|---|---|---|---|---|---|
| | N | Median | Min | Max | N | Median | Min | Max |
| Nausea | 69 | 6.0 | 1 | 83 | 72 | 7.0 | 1 | 79 |
| Dizziness/Vertigo | 55 | 4.0 | 1 | 69 | 79 | 4.0 | 1 | 79 |
| Constipation | 70 | 9.5 | 1 | 94 | 64 | 11.0 | 1 | 63 |
| Somnolence | 65 | 3.0 | 1 | 48 | 45 | 5.0 | 1 | 19 |
| Headache | 27 | 9.0 | 2 | 86 | 38 | 8.5 | 1 | 79 |
| Vomiting | 18 | 13.0 | 3 | 47 | 31 | 5.0 | 1 | 42 |
| Weakness | 24 | 7.0 | 1 | 86 | 31 | 6.0 | 1 | 79 |

| | | | | | | | | |
|---|---|---|---|---|---|---|---|---|
| Table 15: ^{a} adverse events experienced by at least 10% of the patients in at least one of the treatment groups; ^{b}Safety Population | | | | | | | | |

The incidence of headache, vomiting and weakness stabilized after 7 days, whereas dizziness/vertigo, nausea and somnolence required 30 days to stabilize. Reports of constipation continued to increase throughout the study in both groups A similar number of patients In each group (19 in the group taking the inventive composition and 22 In the tramadol BID group) withdrew from treatment because of adverse events.

**Table 16: Most Common Adverse Events ^{a,b}**

| | Inventive Controlled Release Composition | Tramadol BID | p value ^{c} |
|---|---|---|---|
| Adverse Event | N=215 | N=215 | |
| | | | |
| Dizziness or Vertigo | 55 (25.6%) | 79 (36.7%) | 0.0165 |
| Nausea | 70 (32.6%) | 73 (34.0%) | 0.8378 |
| Constipation | 73 (34.0%) | 65 (30.2%) | 0.4697 |
| Somnolence | 65 (302%) | 46 (214%) | 0.0470 |
| Headache | 27 (126%) | 38 (17 7%) | 01779 |
| Vomiting | 18 (8.4%) | 31 (14 4%) | 0.0677 |
| Weakness | 24(11.2%) | 31 (14.4%) | 0.3865 |

| | | | |
|---|---|---|---|
| Table 16: ^{a} adverse events experienced by at least 10% of the patients in at least one of the treatment groups; ^{b} Safety Population; Two-sided Fisher's Exact test | | | |

Eleven (11) serious treatment-emergent adverse events (SAEs) occurred in 11 patients (3 In the group taking the inventive composition; 8 in the tramadol BID group; p = 0 2205) (Table 17) Three of these (cerebrovascular disorder, chest pain and ladder neoplasm) were considered by the investigator to be 'possibly related' to the study medication All SAEs resolved during the study, except for a cerebral ischaemic stroke which occurred in a 67-year old woman and resulted in her death. The investigator determined this event to be 'not related' to the study medication (Table 17).

**Table 17: Listing of Serious Adverse Events^{a}**

| Treatment Group | Event | Duration (days) | Intensity | Relationship | Withdrawn due to AE | Outcome |
|---|---|---|---|---|---|---|
| Inventive Controlled Rebase Composition Tramadol BID | Coronary artery | 7 | Moderate | Not Related | No | Resolved |
| | Ischaemic stroke NOS | 24 | Severe | Not Related | Yes | Death |
| | Essential hypertension | 36 | Mild | Not Related | No | Resolved |
| | Peritoneal adhesions | 6 | Moderate | Not Related | Yes | Resolved |
| | Tibia fracture | 150 | Severe | Not Related | Yes | Resolved |
| | Cerabrovascular disorder | 50 | Severe | Possibly Related | Yes | Resolved |
| | Chest pain | 4 | Moderate | Possibly Related | No | Resolved |
| | Cholelithiasis | 15 | Moderate | Not Related | No | Resolved |
| | Bladder neoplasm | 5 | Moderate | Possibly Related | Yes | Resolved with sequelae |
| | Unstable Angina | 2b | Moderate | Not Related | No | Resolved |
| | Renal colic | 1 | Moderate | Not Related | No | Resolved |

| | | | | | | |
|---|---|---|---|---|---|---|
| Table 17-^{a}Safety Population | | | | | | |

The analgesic efficacy of both treatments was demonstrated with each treatment achieving a 58% reduction on the primary efficacy parameter (percent change in the WOMAC pain sub-scale score from baseline to Week 12) at the same median optimum daily dose in each group (200 mg) Similarity between the treatment groups was confirmed statistically using the 95% confidence interval for the estimated difference in WOMAC pain sub-scale scores This similar efficacy was also demonstrated for the secondary endpoint scores (WOMAC stiffness, physical function, global scores and VAS 24-hour evaluation) Positive ratings by both patients and investigators regarding overall efficacy provided further evidence of the efficacy of both the inventive composition and BID This was followed by continued improvement up until the end of the 12-week study

Seventy-three percent of patients in this study experienced no pain or mild pain immediately prior to taking the morning dose of medication For patients treated with the Inventive composition, this was at the end of the 24-hour dosing interval thus demonstrating the absence of 'end of dose' effects. There was no significant treatment difference between the groups confirming that the inventive composition has a 24-hour duration of action.

The results from the study demonstrated that both formulations were well tolerated, exhibiting the well known, transient and non-serious adverse events profile typically expected with tramadol, including: dizziness/vertigo, nausea, vomiting, constipation, somnolence, headache and weakness. However, the adverse events profile of the inventive controlled release composition showed a clinically significant advantage regarding dizziness/vertigo, vomiting and headache, all of which occurred less frequently and were less severe in the group taking the inventive composition than in the tramadol BID group. Somnolence occurred less frequently in the tramadol BID group.

When compared to the published adverse event profile of tramadol immediate release (IR) (Physicians' Desk Reference, 2002), the adverse event profile of the inventive controlled release formulation is clearly better. Moreover, the difference becomes Increasingly significant with longer treatment duration (in particular, for the incidence of events occurring within 90 days of treatment diainess/Vertigo inventive composition 25% vs. IR 33%; vomiting - inventive composition: 8% vs. IR: 17 %; nausea - inventive composition 32% vs. IR: 40%; constipation - inventive composition: 33% vs. IR: 46%; headache - inventive composition: 13% vs. IR: 32%)

Despite the fact that patients were not allowed to use rescue medication, the total drop out rate In this study (15%) was relatively low compared to the rates reported in the literature for marketed inventive composition (49%) and IR (10% to 56%) formulations (Petrone D. et al "Slowing the titration rate of tramadol reduces the incidence of nausea and/or vomiting: a double-blind, randomised trial." J J Clin Pharm Ther. 1999; 24115-123; Ruoff G. "Slowing the initial titration rate of tramadol improves tolerability." Pharmacotherapy 1999;1 88-93; "Fleischmann RM. et al. "Tramadol for the treatment of joint pain associated with osteoarthritis: a randomized, double-blind, placebo-controlled trial" Curr Ther Res 2001; 62:113-128; and Adler L et al "A comparison of once daily tramadol with normal release tramadol in the treatment of pain in osteoarthritis". J Rhaumatol 2002; 29:10·2196-2199).

The option of treatment with an effective once daily formulation such as the inventive composition which is capable of rapid titration offers patients with chronic pain a significant advantage over faster release formulations The benefit of increased compliance and convenience due to a simplified dosing regimen is relevant to all patients and in particular to elderly patients for whom slmplification of treatment regimens (which often involve multiple medications with multiple dosing regimens) is also a safety issue Since the inventive composition provided efficacy at the same median daily dose (200 mg) as tramadol BID, it should be possible to switch patients already treated with tramadol, to treatment with the inventive composition on a mg per mg basis. This, in combination with the fact that treatment with the Inventive composition offers a clinically favourable adverse events profile in comparison to tramadol BID and immediate release (IR), supports the use of the inventive composition as an analgesic treatment of pain in chronic conditions.

### Example 7

### Analgesic Efficacy and Safety

Adverse effects were evaluated in two studies according to the protocols described below, and results compared to published results obtained with Ultram

A first four-arm study, shown schematically in Figure 17, was carried out to compare the analgesic efficacy and safety of tramadol HCl OAD (100, 200 and 300 mg) versus placebo for the treatment of pain due to osteoarthritis of the knee.

There were approximately 520 patients (100 per active treatment arm and 220 in the placebo arm) enrolled with the aim of having 280 evaluable patients (70 per arm)

This randomized, multi-centre, double-blind, double-dummy, parallel design trial consisted of 3 phases Baseline (Analgesic Washout and Eligibility Assessment), Run-in and Maintenance Patients treated in the Maintenance Phase for up to 12 weeks.

During the Baseline Phase, patients where instructed to discontinue all analgesics and any other medication they may be taking for osteoarthritis, for a minimum of 2 days (or at least 5 drug half-lives). If the level of pain becomes intolerable during the analgesic washout phase, patients were instructed to call the investigator Immediately; the patient was then brought in to see the investigator at the earliest opportunity for evaluation for entry into the treatment phase On the final day of the baseline phase (Visit 1), once the patient was determined to be eligible for the protocol, the patient was randomly assigned to receive one of the four treatments: Tramadol HCl OAD 100 mg or Tramadol HCl OAD 200 mg or Tramadol HCl OAD 300 mg or placebo. No rescue medication was allowed in any of the treatment arms In order to compensate for the corresponding likelihood of a higher drop-out rate among the patients assigned to placebo treatment, 220 patients were randomized to treatment in the placebo arm, while 100 patients were assigned to treatment in each of the active arms.

During the Run-in Phase, patients in the active treatment arms will begin at a dose of 100 mg. For patients in the 200 mg active treatment arm, the dose will be increased after 2 days to 200 mg. Patients in the 300mg active treatment arm, will take 100mg for 2 days, then 200mg for 3 days and finally the dose will be increased to 300 mg Patients In the 100 mg treatment arm will take 100 mg for the duration of the trial Patients in the placebo arm will take placebo for the duration of the trial. All patients will remain in the Run-in Phase for 6days.

During the Maintenance Phase, patients took the Randomized Dose for 12 weeks (84 days)

Efficacy and Safety Evaluations occurred at Visit 1 (Baseline), Visit 2 (M0), Visit 3 (M21), Visit 4 (M42) and Visit 5 (M84)

If a patient discontinued early, he or she was return to the clinic for a Discontinuation Visit. All of the efficacy and safety evaluations was performed at the Discontinuation Visit.

Patients were randomized at Visit 1 to treatment in either the Tramadol HCf OAD 100 mg arm, the Tramadol HCl OAD 200 mg arm, the Tramadol HCl OAD 300 mg arm or the placebo arm

Participants orally self-administered the study medication They took the treatment once daily before breakfast. Patients took 2 tablets each morning. Those in the 100 and 200 mg active treatment arms took 1 active tablet (either 100 mg or 200 mg) and one placebo tablet. Those in the 300 mg arm took two active tablets (100 and 200 mg) Patients assigned to placebo treatment took 2 placebo tablets identical to the 100 and 200 mg active treatments

The medication was supplied in blister packs. The blister packs contained either active and placebo treatment, all active treatment or all placebo treatment depending upon which aim the patient had been randomized to [167.13] The efficacy, safety and clinical benefit of tramadol HCl OAD 100, 200 and 300mg versus placebo were evaluated.

Tramadol formulations currently marketed in the U.S. require at least four times daily dosing. Frequent dosing may affect quality of life and regimen compliance. A once-a-day formulation may address these issues and additionally provide a better efficacy and safety profile.

Males or females aged 40-75 with moderate to severe symptomatic OA of the knee (as per the ACR Criteria) were included. They had to be willing to cease taking any pain medication other than the study medication Patients with known rheumatoid arthritis, other rheumatoid disease or secondary arthritis were excluded; as were those who previously failed Tramadol HCl therapy or discontinued it due to adverse events, who had a history of seizures or who were taking medications which lower the seizure threshold

Primary End Points were as follows: the percentage difference between WOMAC Pain Subscale Score at baseline (R-1) and at the end of the study (Day M 84); the percentage difference between WOMAC Function Subscale Score at baseline (R-1) and at the end of the study (Day M 84; and the average of the Patient Global Rating of Pain at Visits 2 to 5

Secondary Endpoints were as follows the percentage difference between the WOMAC Pain subscale scores at Baseline compared to each of the remaining Visits (2 to 4); the percentage difference between the WOMAC Function subscale scores at Baseline compared to each of the remaining Visits (2 to 4); the multiple-dose effect assessment using the 24-hour Pain questionnaire; the average of the Investigator Global Rating of Pain at Visits 2 to 5; safety evaluations; and the drop out rate.

A second study, shown schematically in Figure 19, was carried out to compare the analgesic efficacy and safety of tramadol hcl once-a-day 100, 200 and 300 mg versus placebo for the treatment of pain due to osteoarthritis of the knee.

Approximately 520 patients (100 per active treatment arm and 220 In the placebo arm) were enrolled with the aim of having 280 evaluable patients (70 per arm).

This randomized, multi-centre, double-blind, double-dummy, parallel design trial consisted of 4 phases: Baseline (Analgesic Washout and Eligibility Assessment). Run-in, Maintenance and Post-Treatment Follow-up. Patients were treated in the Maintenance Phase for up to 12 weeks.

During the Baseline Phase, patients who signed an informed consent were instructed to discontinue all analgesics and any other medication they might be taking for osteoarthritis, for a minimum of 2 days (or at least 5 drug half-lives), If the level of pain becomes intolerable during the analgesic washout phase, patients were instructed to call the investigator immediately; the patient was then brought in to see the Investigator at the earliest opportunity for evaluation for entry into the treatment phase On the final day of the baseline phase (Visit 1), once the patient was determined to be eligible for the protocol, the patient was randomly assigned to receive one of the four treatments Tramadol HCl OAD 100 mg or Tramadol HCl OAD 200 mg or Tramadol HCl OAD 300 mg or placebo. No rescue medication was allowed in any of the treatment arms In order to compensate for the corresponding likelihood of a higher drop-out rate among the patients assigned to placebo treatment, 220 patients were randomized to treatment In the placebo arm, while 100 patients were assigned to treatment In each of the active arms. This was done to permit 70 evaluable patients per arm to complete the study

During the Run-in Phase, patients in the active treatment arms began at a dose of 100 mg, For patients in the 200 mg active treatment arm, the dose was increased after 2 days to 200 mg Patients in the 300 mg active treatment arm took 100 mg for 2 days, then 200 mg for 3 days and finally the dose was increased to 300 mg Patients in the 100 mg treatment arm took 100 mg for the duration of the trial. Patients in the placebo arm took placebo for the duration of the trial. All patients remained in the Run-in Phase for 6 days.

During the Maintenance Phase, patients took the Randomized Dose for 12 weeks (84 days).

Efficacy and Safety Evaluations occured at Visit 1 (Baseline), Visit 2 (M0), Visit 3 (M21), Visit 4 (M42) and Visit 5 (M84).

If a patient discontinued early, he or she was returned to the clinic for a Discontinuation Visit All of the efficacy and safety evaluations were performed at the Discontinuation Visit.

During the Post-Treatment Follow-up Phase, all patients were contacted at 3 and 7 days after their last dose in order to assess for symptoms of withdrawal or dependence. All patients were contacted regardless of whether they discontinued early or completed the study at Visit 5 (Day M84).

Patients were randomized at Visit 1 to treatment in either the Tramadol HCl OAD 100 mg arm, the Tramadol HCl OAD 200 mg arm, the Tramadol HCl OAD 300 mg arm or the placebo arm

Participants orally self-administered the study medication They took the treatment once daily before breakfast Patients took 2 tablets each morning Those in the 100 and 200 mg active treatment arms took 1 active tablet (either 100 mg or 200 mg) and one placebo tablet Those in the 300 mg arm took two active tablets (100 and 200 mg). Patients assigned to placebo treatment took 2 placebo tablets identical to the 100 and 200 mg active treatments.

The medication was supplied in blister packs. The blister packs contained either active and placebo treatment, all active treatment or all placebo treatment depending upon which arm the patient has been randomized to.

The efficacy, safety and clinical benefit of Tramadol HCl OAD 100, 200 and 300mg versus placebo were evaluated.

ramadol formulations currently marketed in the U.S. require at least four times daily dosing. Frequent dosing may affect quality of life and regimen compliance. A once-a-day formulation may address these issues and additionally provide a better efficacy and safety profile.

Males or females aged 40-75 with moderate to severe symptomatic OA of the knee (as per the ACR Criteria) were included. They had to be willing to cease taking any pain medication other than the study medication Patients with known rheumatoid arthritis, other rheumatoid disease or secondary arthritis were excluded; as were those who previously failed Tramadol HCl therapy or discontinued it due to adverse events, who have a history of seizures or who are taking medications which lower the seizure threshold.

Primary End Points were as follows the percentage difference between WOMAC Pain subscale Score at baseline (R-1) and at the end of the study (Day M 84); the percentage difference between WOMAC Function subscale Score at baseline (R-1) and at the end of the study (Day M 84); and the average of the Patient Global Rating of Pain at Visits 2 to 5.

Secondary Endpoints were as follows: the percentage difference between the WOMAC Pain subscale scores at Baseline compared to each of the remaining Visits (2 to 4); the percentage difference between the WOMAC Function subscale scores at Baseline compared to each of the remaining Visits (2 to 4); the multiple-dose effect assessment using the 24-hour Pain questionnaire; the average of the Investigator Global Rating of Pain at Visits 2 to 5; safety evaluations; and drop out rate

The results obtained in the two studies are presented, respectively, in Tables 18 and 19, and graphically in Figures 18 and 20, and for comparison published information on Ultram (product monograph with copyright notice dated 2001) is included for comparison

**Table 18 Comparison of Adverse effects of Tramadol HCL OAD and Ultram¹**

| **Adverse event (%)** | **Treatment** | |
|---|---|---|
| | **Tramadol HCL OAD¹** **(N=338)** | **Ultram²** **(N=427)** |
| Dizziness/Vertigo | 172% | 33% |
| Nausea | 21.9% | 40% |
| Constipation | 133% | 46% |
| Headache | 6.8% | 32% |
| Somnolence | 4.1% | 25% |
| Vomiting | 77% | 17% |
| Pruritus | 5.0% | 11% |
| CNS Stimulation | 33% | 14% |
| Asthenia | 0% | 12% |
| Sweating | 24% | 9% |
| Dyspepsia | 2.1% | 13% |
| Dry Mouth | 3.3% | 10% |
| Diarrhea | 1.8% | 10% |

| | | |
|---|---|---|
| "CNS Stimutation" is a composite of nervousness, anxiety, agitation, tremor, spasticity, euphoria, emotional liability and hallucinations ¹ Ultram Monograph, table 2 (AEs at 90 days) | | |

**Table 19 Comparison of Adverse effects of Tramadol HCL OAD and Ultram**

| **Adverse event (%)** | **Treatment** | |
|---|---|---|
| | **Tramadol HCL OAD** **(N=325)** | **Ultram** **(N=427)** |
| Dizziness/Vertigo | 142% | 33% |
| Nausea | 191% | 40% |
| Constipation | 114% | 46% |
| Headache | 68% | 32% |
| Somnolence | 12.0% | 25% |
| Vomiting | 80% | 17% |
| Pruritus | 8.3% | 11% |
| CNS Stimulation | 43% | 14% |
| Asthenia | 0% | 12% |
| Sweating | 3 4% | 9% |
| Dyspepsia | 12% | 13% |
| Dry Mouth | 4.3% | 10% |
| Diarrhea | 1.5% | 10% |

### Titration

In the European study phase III clinical study described herein, only 8.8% of the patients taking the inventive composition and 10.2% of tramadol BID patients discontinued treatment early as a result of AEs. This suggests that the use of slower release formulations such as the inventive controlled release composition may permit faster titration and lead to a lower incidence of discontinuation of treatment due to adverse events compared to twice daily or IR formulations.

The method of titration of the inventive once daily oral pharmaceutical composition involves as a first step administering to a human patient a unit dose of the inventive composition

If the analgesic effect is not found to be sufficient, the titration is continued by administering to the patient on a subsequent day, an adjusted dose of the inventive once daily formulation Once appropriate analgesic effect is achieved and the final dosage is well tolerated, then treatment of the patient at the final dose, on a once-a-day basis, of the inventive controlled release composition is maintained

The dose range in the phase III European clinical study reported herein (i e. 100 mg, 200 mg, 300 mg and 400 mg daily) was based upon the doses commonly used with various marketed formulations of tramadol During the titration phase of the study each patient was titrated to his or her optimum dose (up to a maximum of 400 mg), which titration lasted between 4 and 10 days. During the titration phase, patients took the 100 mg dosage form for 2 to 3 days. If the 100 mg dosage form was not considered to be that patient's optimum dose, then it was increased to 200 mg at day 3 or 4 and maintained for 1 to 2 days if the 200 mg dose was not considered to be that patients optimum dose, then it was increased to 300 mg on day 5, 6 or 7 If the 300 ing dose was not considered to be that patient's optimum dose, then it was increased to 400 mg on day 7, 8, 9 or 10 The optimum dose was taken throughout the maintenance phase of the study.

In two other clinical studies conducted in the United States and involving the inventive controlled release composition, during the titration phase of the study, each patient was titrated to randomly assigned blinded dose level using double blind, double dummy titration packs. During the titration phase, patients took the 100 mg dosage form for 2 days (that is, days 1 and 2) Patients who were assigned to the 100 mg treatment arm continued to take 100 mg daily for the rest of the study. If the patient was assigned to the 200 mg study arm, the dose was increased to 200 mg on day 3 which dose was then maintained for the rest of the study If the patient was assigned to the 300 mg arm, the same titration up to 200 mg was followed, then on day 5 the dose was increased to 300 mg and then maintained for the rest of the study

A titration period of 2 to 3 days with the inventive controlled release composition is sufficient to reach an optimum dose of 200 mg without an increase in the drop out rate as a result of adverse effects in addition, it is possible to titrate to 300 mg by day 5, while maintaining tolerability, a faster titration than has been seen with any other tramadol formulations The inventive controlled release composition is capable of rapid titration as a result of its favourable adverse event profile compared to other once daily tramadol formulations

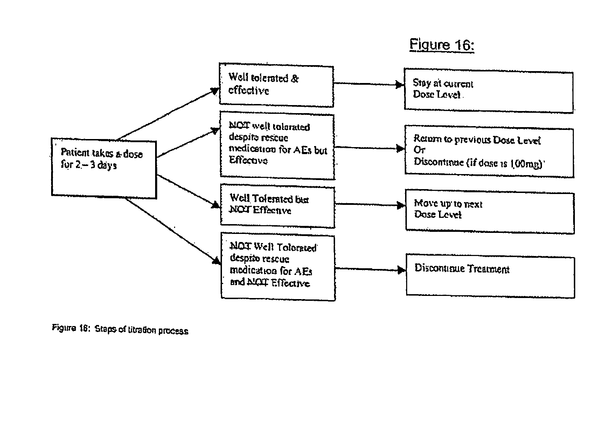

A flow chart is provided at Figure 16 which identifies the possible steps throughout the titration process

The inventive controlled release composition may be packaged into a titration kit to be used for rapid titration of the composition. Such kits may comprise, at a minimum, two 100 mg dosage tablets and one 200 mg if the patient will be titrated to 200 mg as his/her optimum dose. Once the titration is completed, the patient would continue taking the 200 mg dosage daily during maintenance therapy. The tablets required for maintenance therapy are not required to be included in the titration kit

Optionally, the titration kit may contain additional dosage strengths of the inventive controlled release formulations, if a longer titration is desired and if the patient requires a higher dosage strength to achieve effective analgesia. For example, if a patient wishes to titrate up to 400 mg of tramadol, the titration kit would contain additional 200 mg tablets of the inventive controlled release composition, as the patient would take two 200 mg tablets at the same time after having taken the 300 mg dose for a minimum of two days

Optionally, the titration kit may contain instructions for its use. In addition, the titration kit may contain more than the minimum number of tablets required for titration, in the event that a patient wishes to titrate down to a lower dosage in the course of finding his or her optimum dose

The present Invention is not limited in scope by the specific embodiments disclosed in these examples which are intended to illustrate the most preferred embodiments of the invention Indeed, various modifications of the invention or other embodiments which are functionally equivalent to those shown and described herein will become apparent to those skilled in the art and are intended to be covered by the appended claims

A number of references have been Cited, the entire disclosures of which are incorporated herein by reference

Although various examples of combined elements of the Invention have been described, it will also be understood that these are not intended to be exhaustive and features of one embodiment may be combined with those of another, and such other combinations are contemplated to be within the scope of the invention disclosed herein

## Claims

1. A pharmaceutical composition for controlled release of tramadol or a salt thereof, for its use in a once daily oral treatment for pain.

2. The pharmaceutical composition of claim for its use in a once daily oral long-term treatment for chronic pain.

3. The pharmaceutical composition of claim 1 or 2, wherein the composition, upon oral administration, results in an area under the concentration-time curve within 24 hours after administration (AUC0-24) which is comparable to the AUC0-24 resulting from the administration of two doses of the twice daily oral pharmaceutical composition for controlled release of tramadol, taken 12 hours apart.

4. The pharmaceutical composition of any one of claims 1 to 3 comprising 200 mg of tramadol or a salt thereof.

5. The pharmaceutical composition of any one of claims 1 to 3 comprising 300 mg of tramadol or a salt thereof.

6. The pharmaceutical, composition of any one of claims 1 to 3comprising 400 mg of tramadol or a salt thereof.

7. The pharmaceutical composition of claim 4 wherein the minimum mean plasma concentration (Cₘᵢₙ) of the pharmaceutical composition is between 100 ng/mL and 120 ng/mL.

8. The pharmaceutical composition of claim 7, wherein the Cₘᵢₙ is less than 120 ng/mL.

9. The pharmaceutical composition of claim 1 or 2 for its use on each of days I to 3, of about 100 mg of tramadol or a salt thereof; on each of days 4 to 6, of about 200 mg of tramadol or a salt thereof; and on day 7, of about 300 mg of tramadol or a salt thereof.

10. The pharmaceutical composition of claim 1 or 2 for its use on each of days 1 to 3, of about 100 mg of tramadol or a salt thereof; on each of days 4 to 6, of about 200 mg of tramadol or a salt thereof; on each of days 7 to 9, of about 300 mg of tramadol or a salt thereof; and on day 10, of two doses comprising 200 mg of tramadol or a salt thereof.

11. The pharmaceutical composition of claim 1 or 2 for its use on each of days 1 and 2, of about 100 mg of tramadol or a salt thereof; on each of days 3 to 5, of about 200 mg of tramadol or a salt thereof; and on day 6, of about 300 mg of tramadol or a salt thereof.

12. The pharmaceutical composition of claim 1 or 2 for its use on each of days 1 and 2, of about 100 mg of tramadol or a salt thereof; on each of days 3 and 4, of about 200 mg of tramadol or a salt thereof; and on day 5, of about 300 mg of tramadol or a salt thereof.

13. The pharmaceutical composition of claim 1 or 2 for its use on each of days 1 and 2, of about 100 mg of tramadol or a salt thereof; and on day 3, of about 200 mg of tramadol or a salt thereof.

14. The pharmaceutical composition of claim 1 or 2, wherein between 10% and 40% of the agent is released from the formulation between 0 and about 2 hours of measurement, between about 30% and 60% of the agent is released from the formulation between 2 and about 7 hours of measurement, between about 50% and 80% of the agent is released from the formulation between 7 and about 12 hours of measurement, and between about 80% and 100% of the agent is released from the formulation after about 20 hours of measurement.

15. The pharmaceutical composition of claim 1 or 2, wherein the composition, upon initial administration, provides an onset of analgesic effect within 2 hours, which therapeutic effect continues for at least 24 hours after administration and having a dissolution rate in vitro when measured with HPLC-USP apparatus Type 1 at 100 rpm in 50mM sodium phosphate buffer at pH 6.8, from about 5% to about 30% after 1 hour; from about 15% to about 40% after 2 hours; from about 20% to about 50% after 4 hours, from about 30% to about 70% after 8 hours; from about 40% to about 90% after 12 hours; from about 50% to about 100% after 16 hours; from about 60% to about 100% after 24 hours.

16. The pharmaceutical composition of claim 1 or 2, wherein the composition, upon initial administration, provides an onset of analgesic effect within 2 hours, which therapeutic effect continues for at least 24 hours after administration and having a dissolution rate in vitro when measured with HPLC-USP apparatus Type 1 at 100 rpm in 50mM sodium phosphate buffer at pH 6 8, from about 10% to about 25% after 1 hour; from about 15% to about 30% after 2 hours; from about 25% to about 40% after 4 hours, from about 40% to about 55% after 8 hours; from about 60% to about 75% after 12 hours; from about 70% to about 90% after 16 hours; from about 90% to about 100% after 24 hours.

17. The pharmaceutical composition of claim 1 or 2 for its use on day 1, of about 100 mg tramadol; and on day 3, of at least about 200 mg tramadol.

18. The pharmaceutical composition of claim 17 for its use further comprising about 1 00 mg tramadol on day 2.

19. The pharmaceutical composition of claim 17 for its use further comprising at least about 300 mg tramadol on day 5.

20. The pharmaceutical composition of claim 17 for its use further comprising at least about 300 mg tramadol on day 6.

21. The pharmaceutical composition of claim 17 for its use further comprising at least about 300 mg tramadol on day 7.

22. The pharmaceutical composition of claim 17 for its use further comprising at least about 200 mg tramadol on day 4.

23. The pharmaceutical composition of claim 17 for its use on each of days I and 2, of about 100 mg tramadol, and on each day thereafter, of at least about 100 mg tramadol; on at least day 4, of about 200 mg tramadol, and on each day thereafter, of at least about 200 mg tramadol; and on at least day 7, of about 300 mg tramadol, and on each day thereafter, of at least about 300 mg tramadol; wherein an equal daily dosage is administered for at least two consecutive days prior to administering an increased dosage.

24. The pharmaceutical composition of claim 17 for its use on each of days 1 and 2, of about 100 mg tramadol, and on each day thereafter, of at least about 100 mg tramadol; on day 4, of about 200 mg tramadol, and on each day thereafter, of at least about 200 mg tramadol; on at least day 7, of about 300 mg tramadol, and on each day thereafter, of at least about 300 mg tramadol; and on day 8, of about 400 mg tramadol; wherein: for a daily dosage of up to 300 mg, the dosage is administered for at least two consecutive days prior to administering an increased dosage.
